# EUROPEAN PATENT SPECIFICATION

(11) **EP 0 346 831 B1**
(45) Date of publication and mention of the grant of the patent: **22.02.1995**
(21) Application number: 89110689.0
(22) Date of filing: 13.06.1989
(51) Int. Cl.: C07G 11/00, C12P 1/06, C12N 1/20, A61K 35/66

(54) **Antibiotics and process for producing them**
Antibiotika und Verfahren zu deren Herstellung
Antibiotiques et leur procédé de préparation

(30) Priority: 14.06.1988 IT 2095688
(43) Date of publication of application: 20.12.1989
(73) Proprietor: MINISTERO DELL' UNIVERSITA' E DELLA RICERCA SCIENTIFICA E TECNOLOGICA, 00196 Roma (IT)
(72) Inventor: Andriollo, Nunzio, Dr., I-20021 Bollate, Milan (IT); Tolentino, Daniela, Dr., I-20133 Milan (IT); Cassani, Giorgio, Dr., I-20010 Arluno, Milan (IT); Borgonovi, Giorgio, Dr., I-20133 Milan (IT); Vincenti, Marco, Dr., I-10139 Torino (IT); Spera, Silvia, Dr., I-21020 Daverio, Varese (IT); Mirenna, Luigi, I-20139 Milan (IT); Pirali, Giorgio, Dr., I-21047 Saronno, Varese (IT); Confalonieri, Giovanni, Dr., I-20052 Monza, Milan (IT)
(74) Representative: Barz, Peter, Dr.

## Description

The present invention relates to antibiotic substances arbitarily named "AB-011 Antibiotics" and to the main components thereof, i.e. antibiotic AB-011 a and antibiotic AB-011 b.

Furthermore, the present invention relates to the process for preparing said antibiotics, by means of the fermentation of Streptomyces s.p. NCIB 12629, and to their use in the treatment of infective diseases in plants caused by microorganisms susceptible to said antibiotics.

The AB-011 antibiotics are different from the known antibiotics. The term "AB-011 Antibiotics" used in the present invention is meant to define a mixture which comprises all of the components endowed with biological activity, for example antifungal activity, produced by the fermentation of Streptomyces s.p. NCIB 12629 under the conditions specified in the following.

Said active components comprise, but are not limited to, those designated as AB-011 a and AB-01 1 b which can be isolated from the mixture.

It is self-evident that the number and the mutual ratios of the components which form the AB-011 antibiotics may vary as a function of the fermentation conditions and of the bacterial strain used.

Furthermore, it is to be understood that the present invention is not limited to the use of Streptomyces s.p. NCIB 12629, but also comprises the use of either natural or artificial mutants and variants of the above microorganism, provided that they produce the AB-011 antibiotics.

Therefore, the present invention is directed to the AB-011 antibiotics obtainable by means of the controlled cultivation under aerobic conditions of Streptomyces s.p. NCIB 12629, or of an equivalent mutant thereof, in an aqueous nutrient cultivation medium, containing sources of carbon and nitrogen as well as inorganic salts, and subsequent separation of said antibiotics and of the main components thereof, i.e. antibiotics AB-011 a and AB-011 b.

### Physico-Chemical Characteristics of Antibiotic AB-011 a a

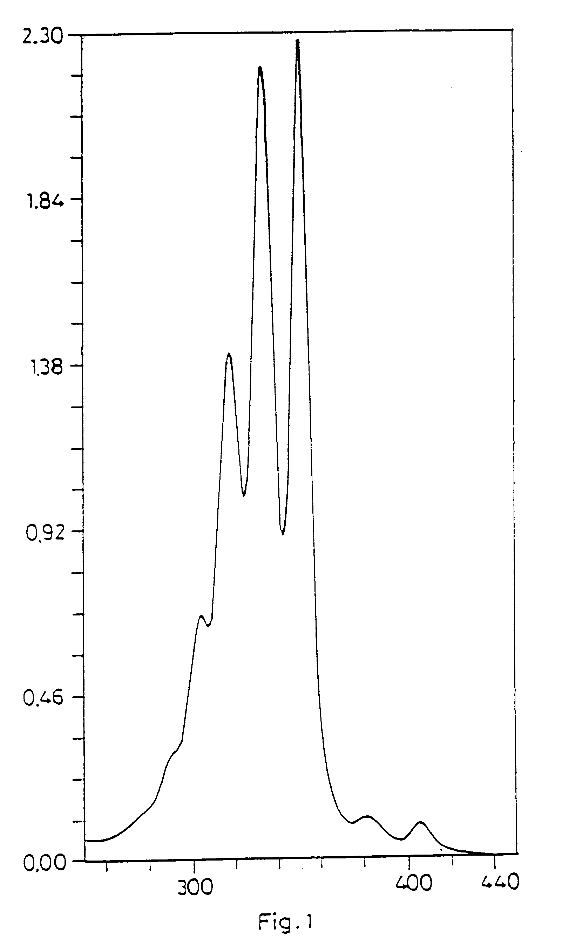

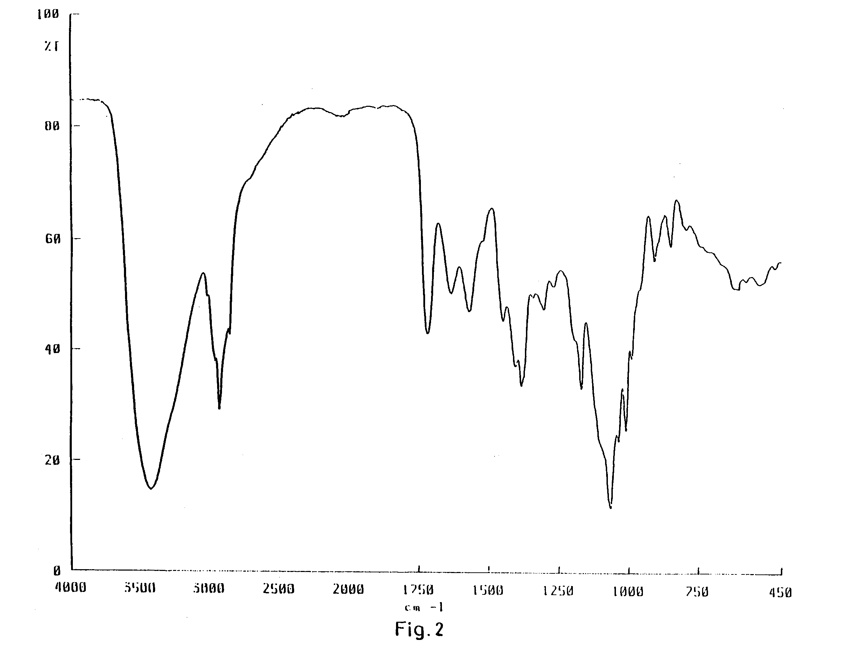

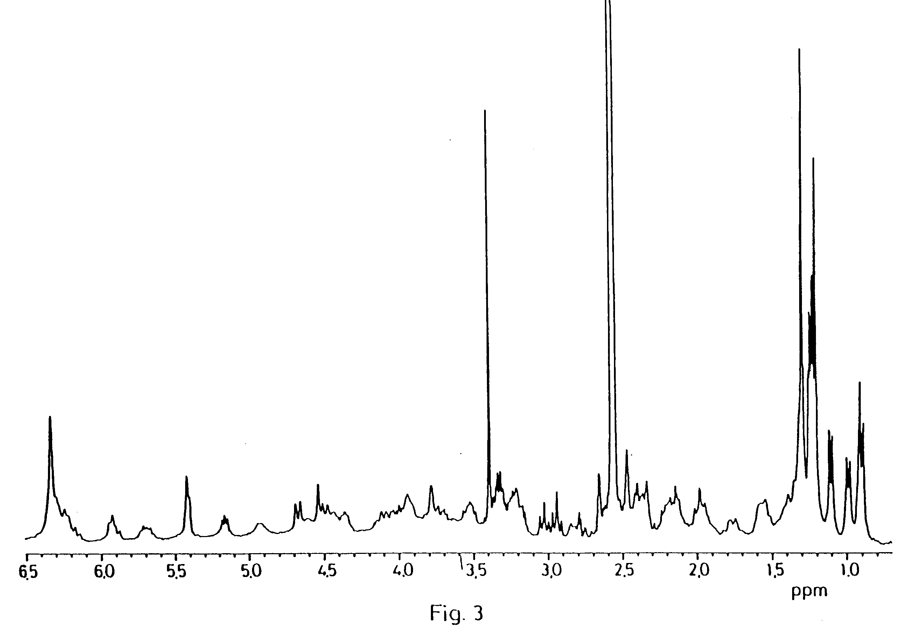

AB-011 a, a component of the AB-011 antibiotics, is a powder of light yellow colour, characterized by:
(a) an approximate elemental analysis, determined on a sample left to stand under vacuum for 2 hours at 40 °C as follows: 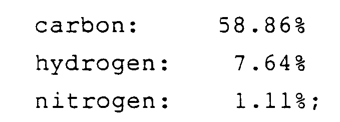 said analysis revealing neither sulfur nor phosphorus;
(b) a molecular weight of about 1,197.65, as determined by FAB-MS spectroscopy, yielding a peak at 1,196.65, corresponding to (M-H)-, under the following operating conditions:
   - Negative ions, FAB, Xe at 9.5 kV
   - Matrix: glycerol
   - Finnigan Mat 8424;
(c) an U.V. absorption spectrum shown in Figure 1 of the attached drawings. Said spectrum shows absorbance maxima of 2.269 at 350.4 nm; 2.197 at 332.6 nm; 1.403 at 317.3 nm; 0.679 at 303.3 nm; 0.118 at 381.1 nm; and 0.097 at 405.6 nm, measured at a concentration of 0.029 mg/ml in methanol;
(d) an I.R. absorption spectrum (KBr pellet) as shown in Figure 2 of the attached drawings, exhibiting the following absorption maxima (cm⁻¹) :
   3421, 2960, 2930, 2855, 2035, 1718, 1634, 1570, 1448, 1403, 1383, 1340, 1302, 1268, 1168, 1063, 1036, 1009, 989, 906, 848, 794, 575, 526, 473;
(e) an ¹H-NMR spectrum recorded on a BRUKER AM 300 MHz spectrometer in hexa-deutero-dimethylsulfoxide (DMSO-d6) and shown in Figure 3, which spectrum exhibits the following signals : (The chemical shifts were indirectly based on TMS = 0.00 ppm (STMS) by using the central peak of hexa-deutero-dimethylsulfoxide at 6TMS = 2.56 ppm as internal reference.)
   6TMS (ppm) : 6.45-6.10* (m, 8H); 5.92 (m, 1 H); 5.69 (m, 1 H); 5.41 (m, 2H); 5.15 (m, 1 H), 4.92 (m, 1 H); 4.77-4.22 (m, 6H); 4.22-3.45* (m, 8-10H); 3.37-3.07* (m, 6-7H); 3.02 (t,1 H); 2.93 (t, 1 H); 2.88-2.72* (m, 3H); 2.45-2.27* (m, 3-4H); 2.27-2.05* (m, 3H); 2.05-1.85* (m, 3H); 1.85-1.66* (m, 1 H); 1.66-1.15* (m, 30-33H); 1.10 (d, 3H); 0.99 (d, 3H); 0.90 (m, 6H).

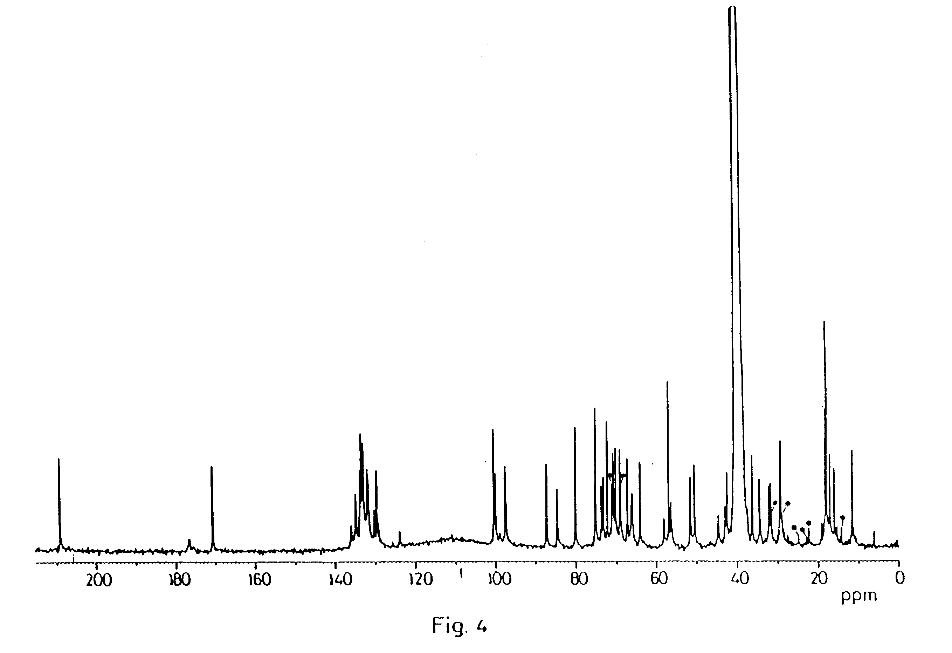

   (The number of hydrogen atoms assigned to the signals marked with an asterisk is only tentative, in that it might be affected by an error.)
(f) a ¹³C-NMR spectrum recorded on a BRUKER AM 300 MHz spectrometer in hexa-deutero-dimethylsulfoxide (DMSO-d6) and shown in Figure 4, which spectrum exhibits the following signals: (The chemical shifts were indirectly based on TMS = 0.00 ppm (δTMS) by using the central peak of hexa-deutero-dimethylsulfoxide at 6TMS = 39.85 ppm as internal reference. The data as to the multiplicity of the signals were obtained by means of DEPT tests at 45, 90 and 135.)
   6TMS (ppm) : 208.6 (s); 176.3 (s); 170.3 (s); 135.8 (d); 134.6 (d); 133.5 (d); 133.2 (d); 132.9 (d); 132.7 (d); 131.7 (d); 131.4 (d); 130.0 (d); 129.5 (d); 100.3 (d); 99.8 (d); 97.4 (s); 97.3 (d); 87.1 (d); 84.4 (d); 79.9 (d); 75.0 (d); 73.5 (d); 73.1 (d); 72.1 (d); 70.7 (d); 70.0 (d); 69.0 (d); 67.1 (d); 66.1 (d); 65.9 (d); 64.0 (d); 58.1 (d); 56.9 (q); 56.3 (d); 51.6 (t); 50.5 (t); 44.7 (t); 43.0 (t); 42.5 (t); 39.7 (d); 39.4 (d); 39.0 (t); 37.5 (t); 36.3 (t); 34.4 (t); 32.1 (t); 31.7 (t); 29.3 (t); 18.0 (q); 17.9 (q); 17.1 (q); 16.0 (q); 11.5 (q).
      (Furthermore, some peaks - marked with an asterisk in Figure 4 - were obtained whose attribution to antibiotic AB-011 a is uncertain in that they are of variable intensity as a function of the origin of the analysed sample: said peaks might originate from decomposition products of the actual product; the following is a list of said peaks:
   δTMS (ppm) : 70.9 (d); 68.9 (d); 31.5 (t); 29.0 (t); 28.8 (t); 28.3 (t); 24.7 (t); 22.8 (q); 22.3 (t); 18.0 (t); 14.2 (q).)
(g) retention coefficients in thin-layer chromatography on silica (Kieselgel) plates of the type 60 F 254 (Merck-Schuchardt) and on reverse-phase silica plates of the type RP-18 F 254 (Merck-Schuchardt) in the following eluent systems and compared to antibiotic AB-011 b (distance covered by eluent: 15 cm):
   eluent A: methanol : 25 mM monobasic potassium phosphate + 7 mM tetramethyl-ammonium chloride in water (8:2);
   eluent B: methanol : acetonitrile : 25 mM monobasic potassium phosphate + 7 mM tetramethyl- ammonium chloride in water (4:4:2);
   eluent C: methanol : 10 mM dibasic ammonium phosphate adjusted to pH 7.5 with phosphoric acid, in water (8:2);
   eluent D: methanol : acetonitrile : 10 mM dibasic ammonium phosphate adjusted to pH 7.5 with phosphoric acid, in water (4:4:2);
   eluent E: ethanol : dioxane : aqueous solution of ammonia (30%) : water (8:1:1:1);
   eluent F: methylene chloride:methanol (17:3).

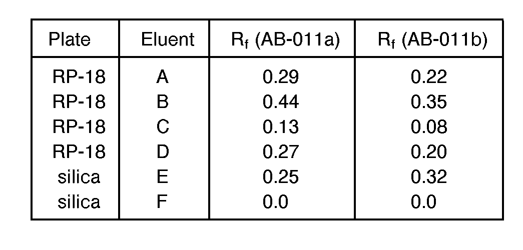

### Visualization:

A. Fluorescence in U.V. light (366 nm)
B. Anisaldehyde - Thin Layer Chromatography - page 205 (T-27 reactant)
C. o-Aminophenol - Thin Layer Chromatography - page 201 (T-11 reactant) Author: Justus G. Kirchner - 2nd Edition Publisher: John Wiley & Sons. (h) a retention time (Rₜ) of about 7 minutes when subjected to reverse-phase HPLC column chromatography under the following conditions:
   Column: Hibar LichroCART Li-Chrosorb@ RP-18 (7 µm) 250 x 4 mm (Merck, Darmstadt, F.R. of Germany)
   Forecolumn: Guard Pak@ RCSS C 18 (Millipore Waters)
   Eluent: methanol: acetonitrile : 25 mM monobasic potassium phosphate + 7 mM tetramethylammonium chloride in water (4:4:2)
   Flow rate: 0.8 ml/minute
   Detector: U.V. at 333 nm
   Temperature: 40°C

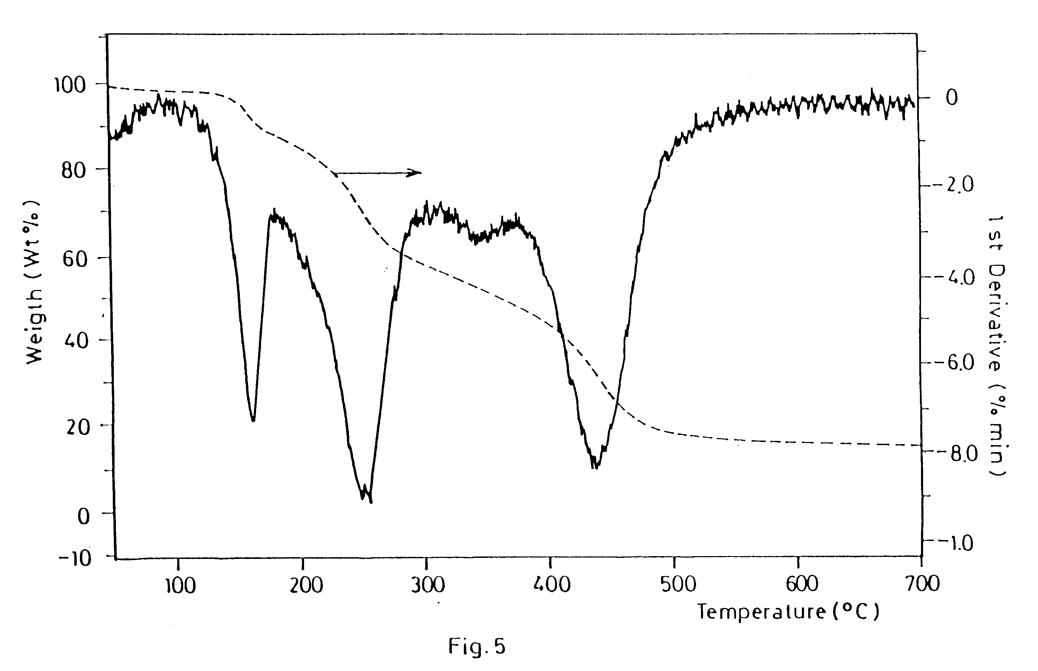

   (Under the same conditions antibiotic AB-011 b was eluted after about 9 minutes.)
(i) a good solubility in dimethylsulfoxide and in (1:1 V/V) ethanol/water or (1:1 V/V) methanol/water mixtures (V/V = volume/volume), poor solubility in water, fairly good solubility in ethanol and methanol;
(j) a diagram derived from a thermogravimetric analysis carried out under nitrogen, with a temperature increase rate of 20 _{°} C/minute within the temperature range of from 30 °C to 700 °C, on a PERKIN-ELMER 7 SERIES Thermal Analysis System, said plot being shown in Figure 5, in which on the abscissa the temperature is given in °C, and on the ordinate the percent weight loss is indicated. In said Figure 5 also the first derivative of the curve is shown.

### Physico-Chemical Characteristics of Antibiotic AB-011 1

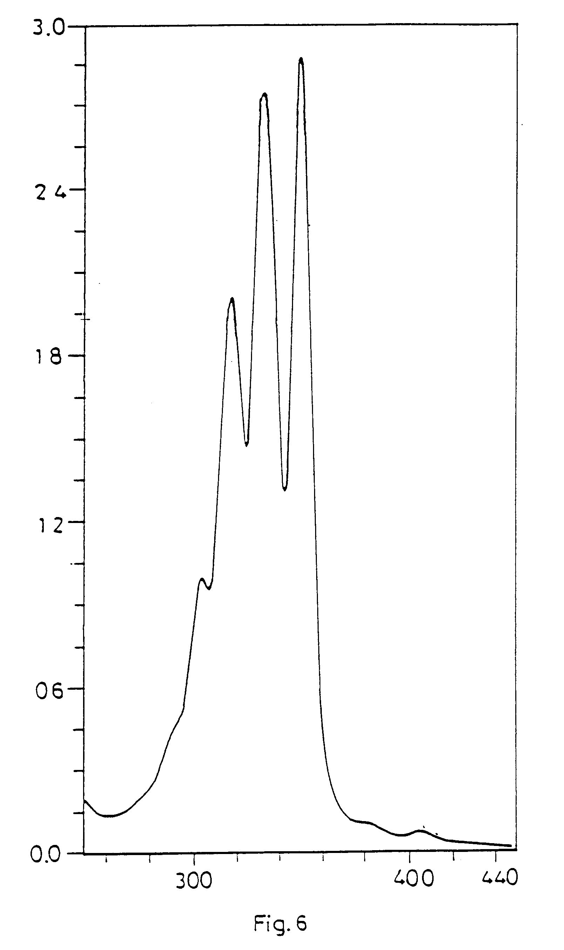

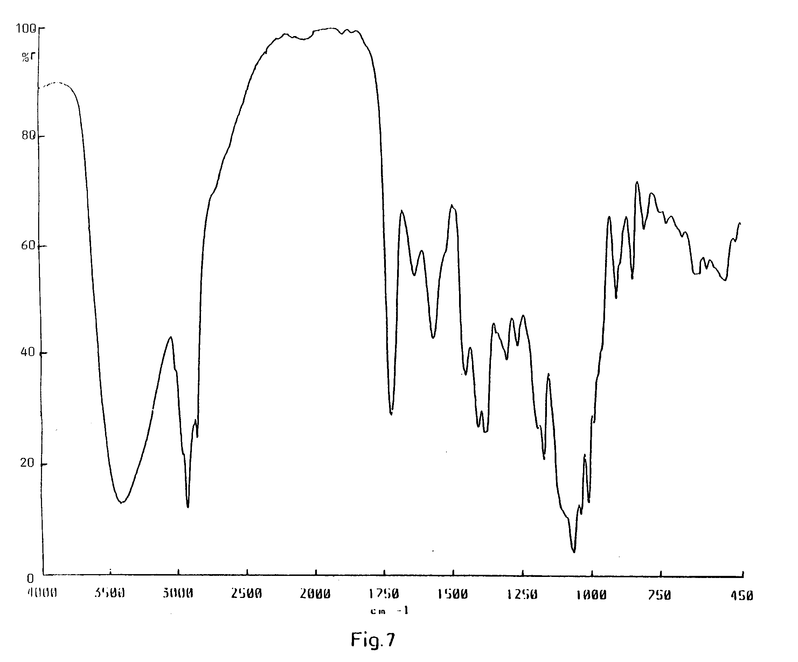

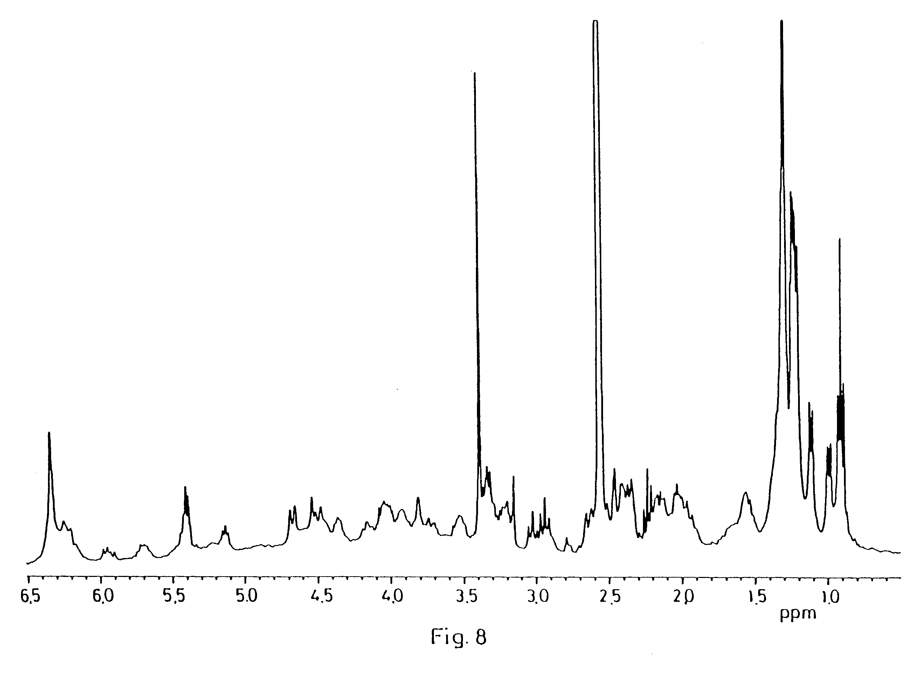

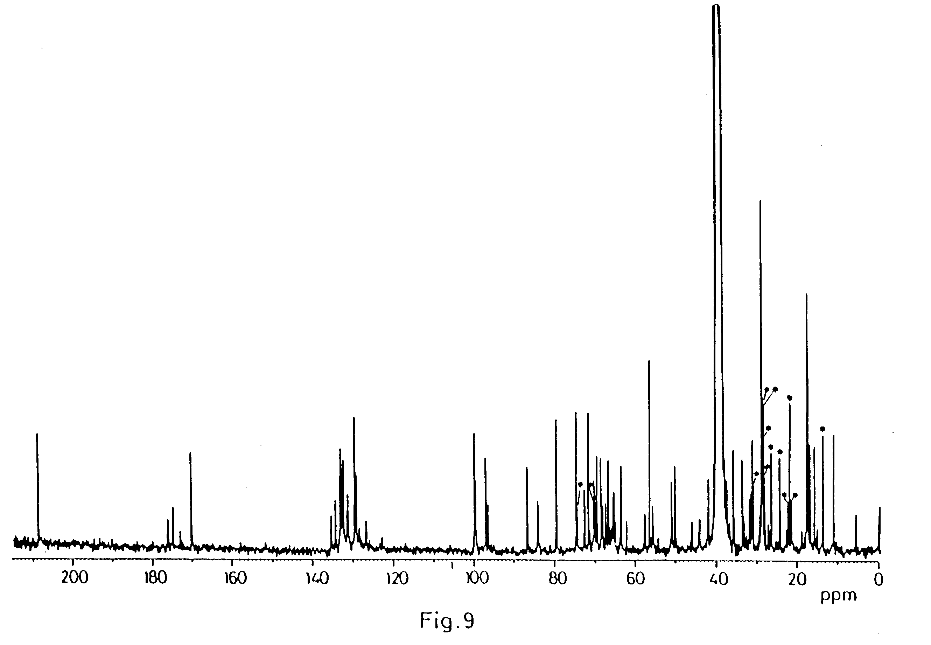

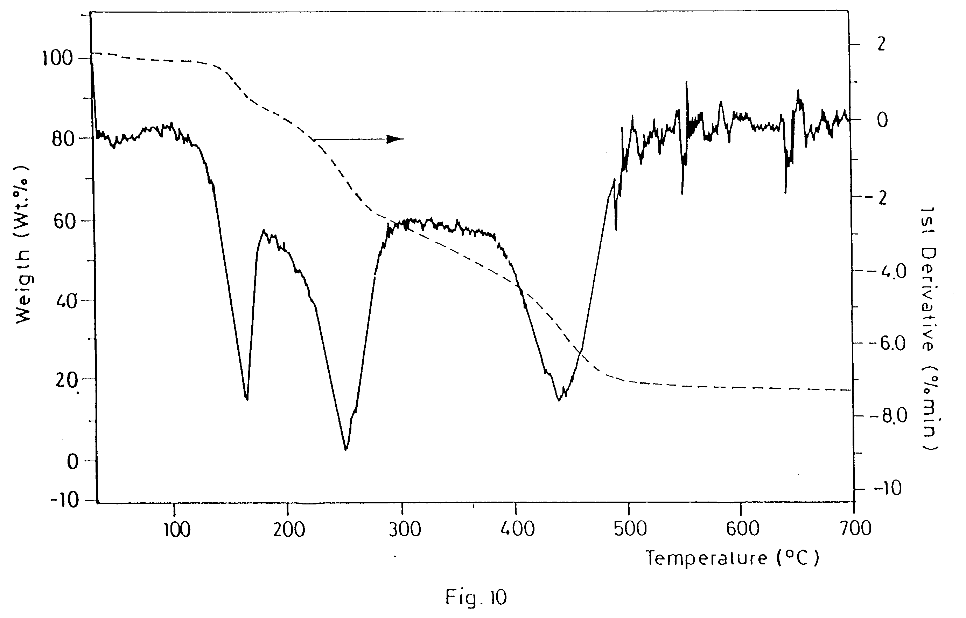

AB-011 b, a component of AB-011 antibiotics, is a powder of deep yellow colour, characterized by:
(a) an approximate elemental analysis, determined on a sample left to stand under vacuum at 40 °C for 2 hours, as follows: 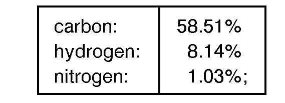 said analysis revealing neither sulfur nor phosphorus;
(b) a molecular weight of about 1,181 as determined by FAB-MS spectroscopy, affording a peak at 1,180, corresponding to (M-H)-,under the following operating conditions:
   - Negative ions, FAB, Xe at 9.5 kV
   - Matrix: glycerol
   - Finnigan Mat 8424;
(c) the U.V. absorption spectrum shown in Figure 6 of the attached drawings.
   Said spectrum shows absorbance maxima of 2.872 at 349.9 nm; 2.747 at 332.4 nm; 1.999 at 316.9 nm; 0.987 at 303.3 nm; measured at a concentration of 0.04 mg/ml in methanol;
(d) an I.R. absorption spectrum (KBr pellet) as shown in Figure 7 of the attached drawings, exhibiting the following absorption maxima (cm-¹):
   3415, 2926, 2855, 2060, 1721, 1634, 1568, 1450, 1406, 1383, 1302, 1264, 1190, 1168, 1063, 1036, 1007, 988, 906, 847, 804, 722, 664, 618, 576, 509, 471, 446;
(e) an ¹H-NMR spectrum recorded on a BRUKER AM 300 MHz spectrometer in hexa-deutero-dimethylsulfoxide (DMSO-d6) and shown in Figure 8 of the attached drawings, said spectrum exhibiting the following signals:
   (The chemical shifts were indirectly based on TMS = 0.00 ppm (δTMS) by using the central peak of hexa-deutero-dimethylsulfoxide at 6TMS = 2.56 ppm as internal reference.)
      δTMS (ppm) : 6.45-6.05* (m, 8H); 5.93 (m, 1 H); 5.70 (m, 1 H); 5.40 (m, 2H); 5.13 (m, 1 H); 4.73-4.30 (m, 6H); 4.27-3.45* (m, 8-9H); 3.37-3.09* (m, 6-7H); 3.02 (t, 1 H); 2.94 (t, 1 H); 2.75-2.60* (m, 1 H); 2.44-2.28* (m, 3H); 2.28-1.81 (m, 7-8H); 1.81-1.47* (m, 5H); 1.47-1.16* (m, 30-33H); 1.11 (d, 3H); 0.99 (d, 3H): 0.91 (m, 6H).
         (The number of hydrogen atoms assigned to the signals marked with an asterisk is only tentative in that it might be affected by error.)
(f) a ¹³C-NMR spectrum recorded on a BRUKER AM 300 MHz spectrometer in hexa-deutero-dimethylsulfoxide (DMSO-d6) and shown in Figure 9 of the attached drawings, said spectrum exhibiting the following signals:
   (The chemical shifts were indirectly based on TMS = 0.00 ppm (δTMS) by using the central peak of hexa-deutero-dimethylsulfoxide at δTMS = 39.85 ppm as internal reference. The data as to the multiplicity of the signals were obtained by means of DEPT tests at 45, 90 and 135.)
      δTMS (ppm) : 208.7 (s); 176.3 (s); 174.9 (s); 170.4 (s); 135.7 (d); 134.7 (d); 133.5 (d); 133.3 (d); 132.9 (d); 132.7 (d); 131.7 (d); 131.5 (d); 130.0 (d); 129.5 (d); 100.3 (d); 100.0 (d); 97.4 (s); 96.9 (d); 87.2 (d); 84.5 (d); 80.0 (d); 75.1 (d); 74.9 (d); 73.1 (d); 72.2 (d); 70.7 (d); 70.0 (d); 69.0 (d); 68.5 (d); 67.8 (d); 67.1 (d); 65.9 (d); 65.7 (d); 63.9 (d); 58.1 (d); 56.9 (q); 56.2 (d); 51.5 (t); 50.7 (t); 46.5 (t); 44.7 (t); 42.5 (t); 39.1 (t); 38.5 (t); 38.0 (t); 36.3 (t); 34.1 (t); 32.3 (t); 31.8 (t); 31.6 (t); 29.3 (t); 18.1 (q); 18.0 (q); 17.9 (q); 17.4 (q); 16.3 (q); 11.6 (q).
         (Furthermore, some peaks - marked with an asterisk in Figure 9 - were obtained, whose attribution to antibiotic AB-011 is uncertain in that they are of variable intensity as a function of the origin of the analysed sample: said peaks might originate from decomposition products of the actual product; the following is a list of said peaks:
      δTMS (ppm) : 74.9 (d); 70.5 (d); 31.4 (t); 29.0 (t); 28.9 (t); 28.8 (t); 28.6 (t); 26.9 (t); 24.8 (t); 22.8 (q); 22.4 (t); 22.0 (t); 14.3 (q).)
(g) retention coefficients (R_{f}) in thin-layer chromatography and retention times (Rₜ) on a reverse-phase HPLC column, respectively, as reported under paragraphs (g) and (h) of the description of the physico-chemical characteristics of antibiotic AB-011 a.
(h) a diagram derived from a thermogravimetric analysis carried out under nitrogen, with a temperature increase rate of 20°C/minute within the temperature range of from 30°C to 700 °C, on a PERKIN-ELMER 7 SERIES Thermal Analysis System, said plot being shown in Figure 10, in which on the abscissa the temperature is given in °C, and on the ordinate the percent weight loss is indicated. In said Figure 10 also the first derivative of the curve is shown.

### Morphology and culture characteristics of microorganism Streptomyces s.p. NCIB 12629

The microorganism was isolated from a sample of soil collected at Varzo (Novara), catalogued under the designation SD18.

A culture of this microorganism was deposited on January 22nd, 1988, in compliance with the Budapest Treaty, with the National Collection of Industrial Bacteria (c/o the National Collection of Industrial and Marine Bacteria Ltd., Torry Research Station, P.O.Box 31, 135 Abbey Road, Aberdeen AB 98 DG, Scotland, United Kingdom), where it was given the access number NCIB 12629.

The morphological characteristics of the strain are listed in Table A (the names of the culture media are those assigned by the International Streptomyces Program).

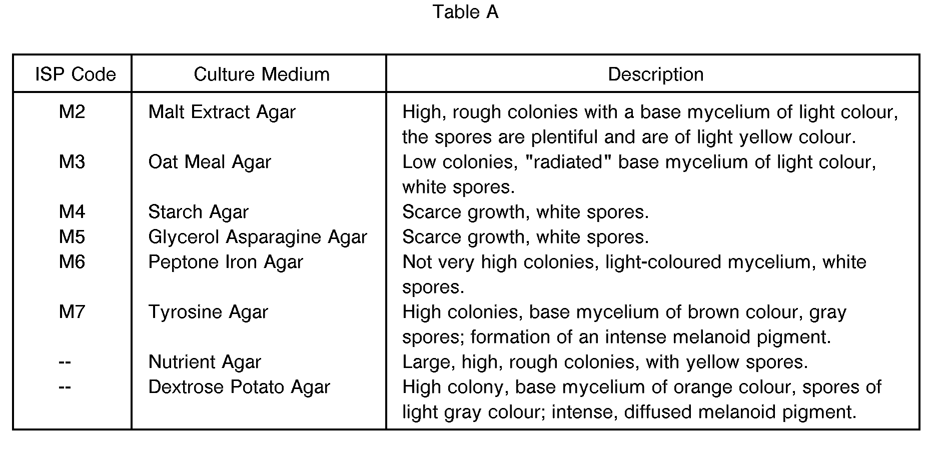

In Table B some characteristics of this strain are reported.

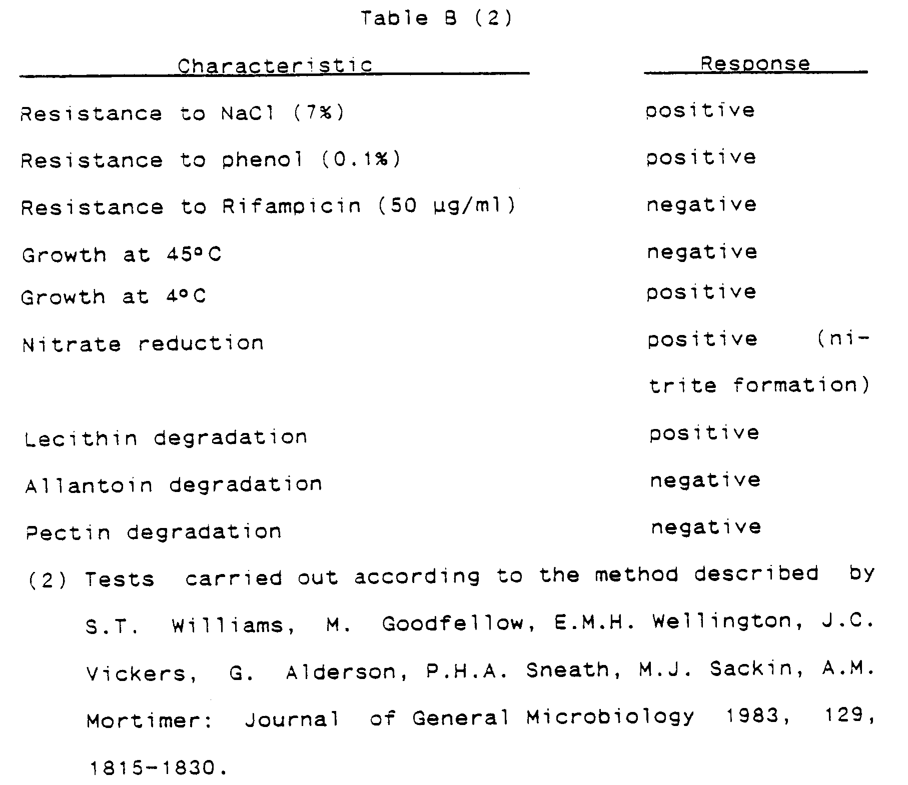

In Table C, the growth of the strain on some organic substances as the only source of carbon is reported.

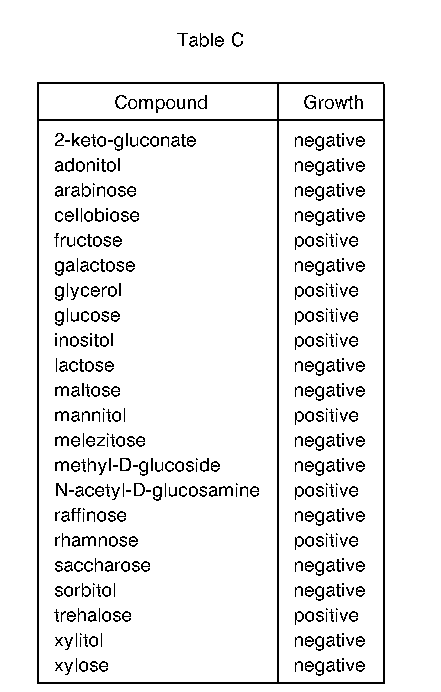

The analysis of the cellular wall of SD18 strain carried out according to the method described by M.P. Starr, H. Stolp, H.G. Truper, A. Ballows, H.G. Shegel (The Prokaryotes - Vol.ll Streptomycetacee - Springer Verlag Ed., 1981) demonstrates the absence of characteristic sugars; thus it confirms that SD18 belongs to the Streptomyces genus.

Like other microorganisms, Streptomyces s.p. NCIB 12629 can undergo mutations.

For example, artificial variants or mutants can be obtained by treatment with various known mutagens, such as X rays or U.V. light, high-frequency waves and chemical substances, like nitrous acid, halogenated alkylamines, nitroso-guandine, camphor, and the like.

All of the variants or mutants, both of natural origin and man-made, which belong to the genus Streptomyces and produce AB-011 antibiotics are considered to be equivalent to Strepromyces s.p. strain NCIB 12629 and are comprised within the scope of the present invention.

### Process of preparation of AB-011 Antibiotics

The process for the preparation of AB-011 antibiotics comprises the cultivation of Streptomyces s.p. NCIB 12629, or of an equivalent mutant thereof, under conditions of controlled aerobic fermentation in an aqueous nutrient medium, and the subsequent separation of said antibiotics by means of per se known methods.

As nutrient culture media or fermentation broths those which are commonly employed for the production of antibiotics can be used; however, some culture media are preferred.

Said cultivation media should contain sources of carbon and nitrogen which can be assimilated by the microorganisms of the genus Streptomyces, and should, furthermore, show low levels of inorganic salts. Additionally, the media should contain traces of those metals which are necessary for the growth and the development of the microorganisms. Said metals may already be present as impurities in the sources of carbon or of protein nitrogen supplied for the bacterial growth or, optionally, they can be added to the culture medium.

As carbon source there can be used carbohydrates which may be of the saccaride type, such as, e.g., glucose or fructose, and of the starch type, or products similar to those types from an industrial point of view, such as, e.g. dextrin, soluble starch, or polyalcohols, such as, e.g. glycerol. Said compounds can be used either individually or in combination.

The concentration of the carbon source in the culture medium generally depends on the type and amount of the other ingredients contained in said medium; anyway, concentrations of from 0.5 to 5% by weight are generally satisfactory. As nitrogen source both proteinic extracts such as, e.g., yeast extract, casein hydrolisate, or peptone, and meals, such as, e.g. soybean meal, or commercially available industrial products for that purpose, such as, e.g., proflo, corn steep liquor or distillers' solubles, can be used.

These compounds can be used individually or in combination, at concentrations in the culture medium of from 0.1% to 4% by weight.

As inorganic salts there can be used, for example, sodium salts, potassium salts, magnesium salts, ammonium salts and calcium salts, such as phosphates, sulfates, chlorides, carbonates and nitrates.

The trace metals can be, e.g., cobalt, manganese, iron, and the like.

Some culture media display a particular ability to stimulate the production of AB-011 antibiotics by Streptomyces s.p. NCIB 12629; among these, for example, the following aqueous formulations can be mentioned, which have been employed in the following preparation Examples.

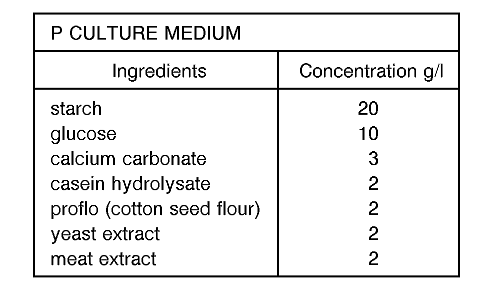

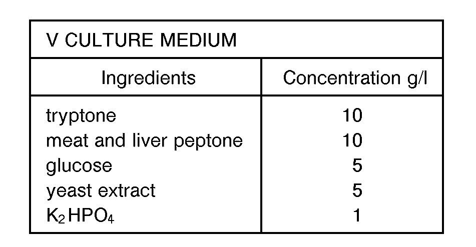

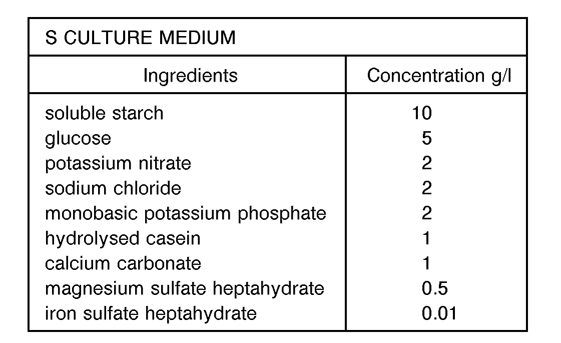

The strain of Streptomyces s.p. NCIB 12629 can be cultivated at temperatures of from 20 _{°} C to 35 °C, preferably of from 25 _{°} C to 30 °C.

The pH value generally is within the range of from about 5 to about 9.

The sterile air which is injected into the culture medium is generally used in such amounts as to maintain in the medium an oxygen concentration equal to, or higher than, 20% of the saturation value.

The production of the antibiotics, during the fermentation, can be monitored by means of antibiotic activity tests on broth samples.

The fermentation is carried out for a time sufficient for obtaining a substantial antibiotic activity; 72-120 hours are generally sufficient for that purpose.

### Separation and purification of the Antibiotics

After the cultivation under the above fermantation conditions, the AB-011 antibiotics and the main components thereof, i.e. antibiotics AB-011 a and AB-011 b, can be separated from the culture broth and subsequently purified by means of methods conventional in the art of fermentation.

Such methods include, e.g., extraction with solvents, precipitation with non-solvents, ultrafiltration, column chromatography, silica-gel chromatography, cellulose chromatography, reverse-phase chromatography, chromatography on non-ionic, macroporous resins, and the like.

The antibiotics produced during the fermentation can be found in the culture broth and/or in the mycelium mass.

A preferred method for recovering the AB-011 antibiotics consists in filtering off the mycelium mass from the culture broth, subjecting the mycelium thus separated to an extraction with acetone or methanol and concentrating the extract under vacuum until complete removal of the solvent. Thereby an aqueous suspension is obtained, while suspension is combined with the culture broth.

The resulting solution containing the AB-011 antibiotics is filtered through fibreglass-paper filters and is then percolated on a column of a non-ionic polystyrene resin, such as, e.g., XAD-2 (Rohm & Haas Co.), which resin adsorbs the AB-011 antibiotics.

The resin is then washed with two volumes, based on its bed, of water, and thereafter is eluted with three volumes, also based on its bed, of an 8:2 (V/V) mixture of acetone:water.

The fractions containing the AB-011 antibiotics, identified by means of biological activity tests on Botrytis, are combined and are then concentrated to dryness under vacuum, affording a crude product containing the AB-011 antibiotics, substantially composed of AB-011 a and AB-011 b.

Both pure AB-011 a and pure AB-01 1 b are then isolated from the crude product by means of reverse phase chromatography, employing a column packed with silica (MATREXO Silica C18 marketed by Amicon Europe, Lausanne, Switzerland) with an eluent system formed by an eluent "A" consisting of water containing 25 millimol per litre of KH₂ P0₄ and 7 millimol per litre of tetramethylammonium chloride, and by an eluent "B" consisting of methanol, using a linear gradient of from 50% up to 80% of eluent "B" in eluent "A".

The fractions which contain antibiotic AB-011 a in pure form and antibiotic AB-01 1 b in pure form are separately concentrated under vacuum until complete removal of methanol. By cooling the remaining aqueous solutions down to 2 _{°} C, the precipitation of AB-011 a and AB-011 b, resp. - which are centrifuged off - is achieved.

AB-011 a and AB-01 1 b separated from their respective solutions as described above are suspended in water and centrifuged again and, after removing the supernatant solutions and drying under vacuum at 40 °C for 2 hours, pure antibiotic AB-011 a and pure antibiotic AB-01 1 b antibiotic, resp., are obtained.

### Biological activity

The AB-011 antibiotics, and the components thereof, i.e., AB-011 a and AB-011 b, are endowed with antimicrobial activity and, particularly, antifungal activity.

Their antifungal activity is particularly high against phytopathogenic fungi which infest herbaceous, arboricultural, industrial and horticultural cultivations.

The antimicrobial activities, both in vitro and in vivo, of AB-011 antibiotics were determined by means of the methods described in the following.

### "in vitro" Activity Test

The antimicrobial activity of AB-011 antibiotics was determined by the usual methods, by means of suitable dilutions in liquid growth medium.

In Table D, the minimum inhibiting concentrations are set forth.

For phytopathogenous fungi, the minimum concentration of the AB-011 antibiotics which, under controlled conditions in agarized medium, caused a reduction in mycellar growth of 90% with respect to the control, was determined.

The corresponding results obtained for yeast (Candida albicans) and bacteria (Sarcina lutea) were 3.5 and 0.3 µg/ml, respectively.

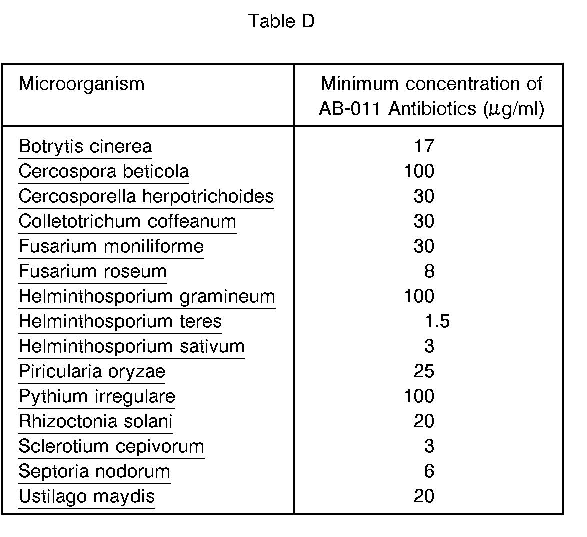

### Fungicidal activity "in vivo"

The fungicidal activity in vivo was measured by the following method. The AB-011 antibiotics in water- acetone solution (20% V/V) were sprayed onto the lower sides of leaves of plants grown in pots inside a conditioned room.

One day later, an inoculum of the tested fungus was sprayed on the upper sides of the plant leaves. These plants were then kept under incubation conditions inside a conditioned room for about eight days. At the end of said time, the seriousness of the infection was evaluated by means of an evaluation scale ranging from 100 (= healthy plant) down to 0 (= completely infected plant).

The data as to the preventive activity in vivo is reported in Table E.

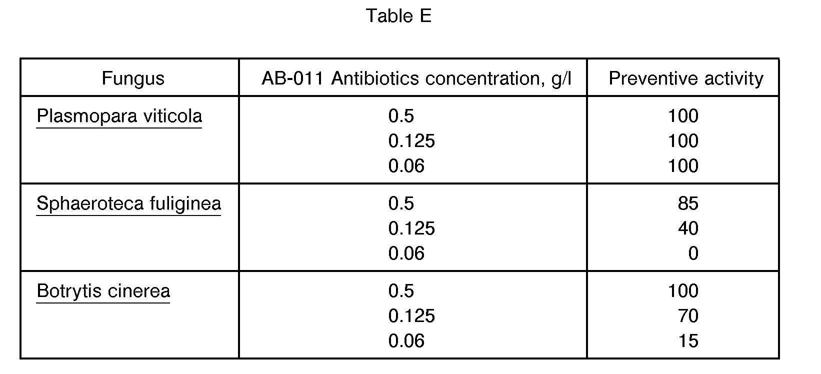

Strictly analogous results of antifungal activity were obtained when the individual antibiotics AB-011 a and AB-011 b were used.

For their practical application, both in agriculture and in other sectors of use, the antibiotics according to the present invention are preferably employed in the form of suitable compositions.

These compositions contain, beside an antibiotic according to the present invention as their active principle, inert solid carriers (e.g., kaolin, silica, talc, attapulgite, diatomaceous earth, etc.), or inert liquid carriers (organic solvents, vegetable or mineral oils, water and mixtures thereof), and possibly other additives which are conventionally used in the art of formulations, such as surfactants, suspending agents, dispersants and wetting agents.

In case of particular application requirements, or in order to expand the range of action of the compositions, other active ingredients, such as other insecticides, herbicides, fungicides, or fertilizers can be added to the present compositions.

The applied doses vary as a function of different factors, such as the type and the degree of infestation, the type of composition used and climatic and environmental factors.

For practical use in agriculture, doses of the present antibiotics of from 10 to 500 g/ha yield satisfactory results.

The following examples serve to illustrate the present invention without limiting it.

### EXAMPLE 1

### Fermentation of Streptomyces s.p. NCIB 12629 strain

An ampoul containing 5 ml of the culture of Streptomyces s.p. NCIB 12629 in the above medium "V" (stored in glycerol at 10% and at -20 °C) was used to inoculate 150 ml of medium "V". Said medium was then incubated for 72 hours on a rotary shaker (150 rpm) at 28 _{°} C.

The resulting culture was used to inoculate a fermenter (volume 10 litres) containing 7 litres of medium "S" to which 0.01 g/I of Tween@ 2000 were added as antifoaming agent, under the following conditions: temperature 29 _{°} C, air flow rate 120 litres/hour, rate of stirring 320 rpm, fermentation time 96 hours.

The resulting fermentation broth was filtered through paper and the mycelium was separated. For the subsequent purification operations a total volume of 28 I, obtained from four fermentations carried out under the above conditions, was used.

### Separation of AB-011 Antibiotics

28 litres of fermentation broth were filtered through a Whatman GF/D fibreglas filter and the separated mycelium was then extracted with acetone.

The acetone extract was concentrated under vacuum until the solvent was completely removed and the residual aqueous solution was combined with the previously filtered broth.

The resulting solution was ultrafiltered through a GR-61 membrane (cut-off 20,000), until 3.2 litres remained in the retained fraction.

The retained fraction was then diluted to 25 litres with water, and was ultrafiltered again, under the same conditions, whereby a volume of permeate of 20.5 litres was obtained. The second retained fraction (4.0 litres), was scarcely active in the activity test (the activity was determined by measuring the Botrytis growth inhibition halo over agar) and therefore was discarded.

The first ultrafiltrate of 25 litres was concentrated on a GR-90 membrane (cut-off 2000), until 3.6 I remained in the retained fraction, while the ultrafiltrate (21.0 I) was scarcely active on Botrytis and was discarded. The retained fraction contained 60% of the initial activity.

The second ultrafiltration permeate (20.5 I) was concentrated on a GR-90 membrane (cut-off 2000) in the same way as described above, and the residual 3.0 litres contained about 30% of the initial activity.

The GR-61 and GR-90 membranes belong to the types sold by DDS-Nakskov (Danmark) and were used on a Lab-20 module also marketed by said company.

The two retained fractions (3.6 I + 3.0 I) were combined and percolated on a column (diameter 45 mm; height of resin bed 30 cm) of non-ionic polystyrene resin XAD-2 (Rohm & Haas Co.), which adsorbed the AB-011 antibiotics. During the percolation, an inlet flow rate of 100 ml/hour was maintained. The resin subsequently was washed with two volumes, based on its bed, of water, and was then eluted with 3 volumes, also based on its bed, of an (8:2 V/V) acetone/water mixture.

The fractions which contained AB-011 antibiotics, identified by means of biological tests carried out on Boytrytis, were combined and concentrated to dryness under vacuum, affording a crude material containing AB-011 antibiotics especially composed of AB-011 a and AB-011 b.

The crude product was taken up in 300 ml of methanol, to which 300 ml of water were subsequently added. Pure AB-011 a and pure AB-01 1 b were then isolated from the crude material by means of a reverse-phase chromatography, using a column filled with 350 g of silica (MATREXO Silica C18, Amicon Europe, Lausanne, Switzerland) and an eluent system formed by eluent "A" (composed of water containing 25 mM monobasic potassium phosphate 7 mM tetramethyl-ammonium chloride) and eluent "B" (containing methanol), employing a gradient according to the following Table:

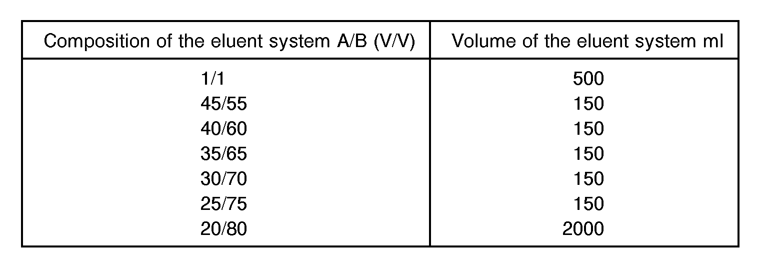

Pure AB-01 1 a was collected in a volume of 320 ml in the elution range between 3000 and 3320 ml, while AB-011 b was collected in a volume of 700 ml in the elution range between 3700 and 4400 ml.

The fractions thus obtained were evaporated under vacuum until methanol was completely removed and then were left to stand for 24 hours at 4°C. From these solutions, AB-011 a and AB-011 b, respectively precipitated and were collected by centrifugation; then they were suspended again in water and centrifuged.

After drying, 70 mg of antibiotic AB-011 a, as a light yellow-coloured powder, and 20 mg of antibiotic AB-011 b, as a deep yellow coloured powder, were obtained. The physico-chemical characteristics of the products have been indicated above.

### EXAMPLE 2

### Fermentation of Streptomyces s.p. NCIB 12629

A freeze-dried ampoul of Streptomyces s.p., strain NCIB 12629, was opened under aseptic conditions and rehydrated with sterile distilled water. The resulting suspension was used to inoculate a 500 ml Erlenmeyer flask containing 100 ml of culture medium "P", described above, which was then incubated for 90 hours on a rotary shaker (180 rpm) at 28 °C.

At the end of this time, the culture broth was centrifuged in order to separate it from the mycelium, and was then used for the biological tests.

### EXAMPLE 3

### Fermentation of Streptomyces s.p. NCIB 12629

A freeze-dried ampoul of Streptomyces s.p., strain NCIB 12629, was rehydrated and used to inoculate the culture medium "P", as described in Example 2.

The culture was incubated for 72 hours on a rotary shaker (180 rpm) at 28 °C.

5 ml of the obtained culture were used to inoculate a 500 ml Erlenmeyer flask containing 100 ml of culture medium "S" (SCM), described above, which was then incubated for 120 hours on a rotary shaker (180 rpm) at 28 _{°} C.

At the end of this time, the culture broth was treated and used in the biological tests in the manner described in Example 2.

### EXAMPLE 4

### Fermentation of Streptomyces s.p. NCIB 12629

A 100 ml Erlenmeyer flask containing 25 ml of culture medium "P" (PMB) as described above was inoculated with a portion of a colony of the Streptomyces s.p. strain NCIB 12629, drawn under aseptic conditions from a plate, or from a slant or agarized "P" medium.

This culture then was incubated for 72 hours on a rotary shaker (180 rpm) at 28 °C. Thereafter the incubated culture was inoculated in a 100 ml Erlenmeyer flask containing 20 ml of the "P" medium up to a concentration of 5% and the whole mixture was incubated for 120 hours under the conditions given in Example 2.

At the end of this time, the culture broth was treated in the same way as described in Example 2 and was subjected to the biological tests.

## Claims (Claims for the following Contracting State(s) : AT, BE, CH, DE, FR, GB, LI, NL, SE)

1. Antibiotic AB-011 a, a solid substance characterized by:
(a) the following approximate elemental analysis: 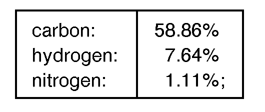
(b) a molecular weight of about 1,197.65;
(c) UV-absorbance maxima of:
2.269 at 350.4 nm; 2.197 at 332.6 nm; 1.403 at 317.3 nm; 0.679 at 303.3 nm; 0.118 at 381.1 nm and 0.097 at 405.6 nm at a concentration of 0.029 mg/ml in methanol;
(d) IR-absorbance maxima at 3421, 2960, 2930, 2855, 2035, 1718, 1634, 1570, 1448, 1403, 1383, 1340, 1302, 1268, 1168, 1063, 1036, 1009, 989, 906, 848, 794, 575, 526 and 473 cm-¹;
(e) main peaks in its ¹H-NMR spectrum at δTMS (ppm) 6.45-6.10* (m, 8H); 5.92 (m, 1 H); 5.69 (m, 1 H); 5.41 (m, 2H); 5.15 (m, 1 H), 4.92 (m, 1 H); 4.77-4.22 (m, 6H); 4.22-3.45* (m, 8-10H); 3.37-3.07* (m, 6-7H); 3.02 (t,1 H); 2.93 (t, 1 H); 2.88-2.72* (m, 3H); 2.45-2.27* (m, 3-4H); 2.27-2.05* (m, 3H); 2.05-1.85* (m, 3H); 1.85-1.66* (m, 1H); 1.66-1.15* (m, 30-33H); 1.10 (d, 3H); 0.99 (d, 3H); 0.90 (m, 6H);
(f) main peaks in its ¹³C-NMR spectrum at
δTMS (ppm) : 208.6 (s); 176.3 (s); 170.3 (s); 135.8 (d); 134.6 (d); 133.5 (d); 133.2 (d); 132.9 (d); 132.7 (d); 131.7 (d); 131.4 (d); 130.0 (d); 129.5 (d); 100.3 (d); 99.8 (d); 97.4 (s); 97.3 (d); 87.1 (d); 84.4 (d); 79.9 (d); 75.0 (d); 73.5 (d); 73.1 (d); 72.1 (d); 70.7 (d); 70.0 (d); 69.0 (d); 67.1 (d); 66.1 (d); 65.9 (d); 64.0 (d); 58.1 (d); 56.9 (q); 56.3 (d); 51.6 (t); 50.5 (t); 44.7 (t); 43.0 (t); 42.5 (t); 39.7 (d); 39.4 (d); 39.0 (t); 37.5 (t); 36.3 (t); 34.4 (t); 32.1 (t); 31.7 (t); 29.3 (t); 18.0 (q); 17.9 (q); 17.1 (q); 16.0 (q); 11.5 (q);
(g) Rₜ values obtained by thin-layer chromatography (TLC) on plates of the type 60F 254 (Merck-Schuchardt) of:
0.25 in an ethanol/dioxane/30% aqueous ammonia/water (8:1:1:1) mixture;
0.0 in a methylene chloride/methanol (17:3) mixture; and
R_{f} values obtained by reverse-phase chromatography on plates of the type RP-18F 254 (Merck-Schuchardt) of:
0.29 in a methanol/aqueous solution containing 25 mM monobasic potassium phosphate + 7 mM tetramethylammonium chloride (8:2) mixture;
0.44 in a methanol/acetonitrile/aqueous solution containing 25 mM monobasic potassium phosphate + 7 mM tetramethyl ammonium chloride (4:4:2) mixture;
0.13 in a methanol/10 mM aqueous solution of monobasic ammonium phosphate adjusted with phosphoric acid to pH 7.5 (8:2) mixture;
0.44 in a methanol/acetonitrile/10 mM aqueous solution of monobasic ammonium phosphate adjusted with phosphoric acid to pH 7.5 (4:4:2) mixture;
(h) a retention time (Rₜ) of about 7 minutes in reverse-phase HPLC on a Hibar Li-ChroCARTO Li-Chrosorb RP-18 column (forecolumn: Guard Pak@ RCSS C 18) when eluting with a methanol/acetonitrile/aqueous solution containing 25 mM of monobasic potassium phosphate + 7 mM tetramethyl-ammonium chloride (4:4:2) mixture at a flow rate of 0.8 ml/minute and at 40 °C;
(i) high solubility in dimethylsulfoxide and ethanol/water (1:1 V/V) or methanol/water (1:1 V/V) mixtures, scarce solubility in water and fairly high solubility in ethanol and methanol.

2. Antibiotic AB-011 b, a solid substance characterized by:
(a) the following approximate elemental analysis: 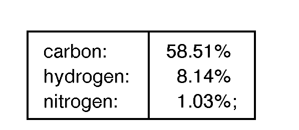
(b) a molecular weight of about 1,181;
(c) UV-absorbance maxima of:
2.872 at 349.9 nm; 2.747 at 332.4 nm; 1.999 at 316.9 nm; 0.987 at 303.3 nm; at a concentration of 0.04 mg/ml in methanol;
(d) IR-absorbance maxima at:
3415, 2926, 2855, 2060, 1721, 1634, 1568, 1450, 1406, 1383, 1302, 1264, 1190, 1168, 1063, 1036, 1007, 988, 906, 847, 804, 722, 664, 618, 576, 509, 471, 446 cm-¹;
(e) main peaks in its ¹H-NMR spectrum at:
δTMS (ppm) : 6.45-6.05 (m, 8H); 5.93 (m, 1 H); 5.70 (m, 1 H); 5.40 (m, 2H); 5.13 (m, 1 H); 4.73-4.30 (m, 6H); 4.27-3.45* (m, 8-9H); 3.37-3.09* (m, 6-7H); 3.02 (t, 1 H); 2.94 (t, 1 H); 2.75-2.60* (m, 1 H); 2.44-2.28* (m, 3H); 2.28-1.81'(m, 7-8H); 1.81-1.47* (m, 5H); 1.47-1.16* (m, 30-33H). 1.1 (d, 3H). 0.99 (d, 3H), 0.91 (m. 6H);
(f) main peaks in its ¹³C-NMR spectrum at
δTMS (ppm) : 208.7 (s); 176.3 (s); 174.9 (s). 170.4 (s); 135.7 (d); 134.7 (d); 133.5 (d); 133.3 (d); 132.9 (d); 132.7 (d); 131.7 (d); 131.5 (d); 130.0 (d); 129.5 (d); 103.3 (d); 100.0 (d); 97.4 (s); 96.9 (s); 87.2 (d); 84.5 (d); 80.0 (d); 75.1 (d); 74.9 (d); 73.1 (d); 72.2 (d); 70.7 (d); 70.0 (d); 69.0 (d); 68.5 (d); 67.8 (d); 67.1 (d); 65.9 (d); 65.7 (d); 63.9 (d); 58.1 (d); 56.9 (q); 56.2 (d); 51.5 (t); 50.7 (t); 46.5 (t); 44.7 (t); 42.5 (t); 39.1 (t); 38.5 (t); 38.0 (t); 36.3 (t); 34.1 (t); 32.3 (t); 31.8 (t); 31.6 (t); 29.3 (t); 18.1 (q); 18.0 (q); 17.9 (q); 17.4 (q); 16.3 (q); 11.6 (q);
(g) Rₜ values obtained by thin-layer chromatography (TLC) on plates of the type 60F 254 (Merck-Schuchardt) of:
0.32 in an ethanol/dioxane/30% aqueous ammonia/water (8:1:1:1) mixture;
0.00 in a methylene chloride/methanol (17:3) mixture; and
Rₜ values obtained by reverse-phase chromatography on plates of the type RP-18F 254 (Merck-Schuchardt) of:
0.22 in a methanol/aqueous solution containing 25 mM monobasic potassium phosphate + 7 mM tetramethyl-ammonium chloride (8:2) mixture;
0.35 in a methanol/acetonitrile/aqueous solution containing 25 mM monobasic potassium phosphate + 7 mM tetramethyl-ammonium chloride (4:4:2) mixture;
0.08 in a methanol/10 mM aqueous solution of monobasic ammonium phosphate adjusted with phosphoric acid to pH 7.5 (8:2) mixture;
0.20 in a methanol/acetonitrile/10 mM aqueous solution of monobasic ammonium phosphate adjusted with phosphoric acid to pH 7.5 (4:4:2) mixture;
(h) a retention time (Rₜ) of about 9 minutes in reverse-phase HPLC on a Hibar Li-ChroCART Li-Chrosorb@ RP18 column (forecolumn: Guard Pak@ RCSS C 18) when eluting with a methanol/acetonitrile/aqueous solution containing 25 mM monobasic potassium phosphate +7 mM tetramethyl-ammonium chloride (4:4:2) mixture at a flow rate of 0.8 ml/minute and at 40 _{°} C.

3. AB-011 Antibiotics, obtainable by means of the controlled cultivation under aerobic conditions of Streptomyces s.p. NCIB 12629, or of an equivalent mutant thereof, in an aqueous nutrient cultivation medium containing sources of carbon and nitrogen and inorganic salts, said antibiotics being substantially composed of antibiotic AB-011 a and antibiotic AB-011 b as defined in claims 1 and 2.

4. Process for the preparation of AB-011 antibiotics, comprising the cultivation of Streptomyces s.p. NCIB 12629, or of a corresponding mutant thereof, under conditions of controlled aerobic fermentation in an aqueous nutrient medium containing assimilatable sources of carbon and nitrogen as well as inorganic salts, until antibiotic activity is obtained, the subsequent recovery of said antibiotics by means of per se known methods, and, optionally, the separation of the main components, antibiotic AB-011 a and antibiotic AB-011 b as defined in claim 1 and 2.

5. Process according to claim 4, in which the fermentation is carried out at a temperature of from 25 _{°} C to 30_{°}C.

6. Process according to any one of claims 4 and 5, in which the fermentation is carried out at a pH within the range of from 5 to 9.

7. Process according to any one of claims 4 to 6, in which the AB-011 antibiotics are isolated from the fermentation broth by means of filtration and subsequent use of chromatographic techniques.

8. Process according to any one of claims 4 to 7, in which the antibiotics AB-011 a and AB-01 1 b are isolated by reverse-phase chromatography on a silica-gel column using a linear elution gradient of from 50% to 80% of methanol in the corresponding mixture with water containing 25 mM/I of KH₂P0₄ and 7 mM/I of (CH₃)₄ NCI.

9. Microorganism Streptomyces s.p.,NCIB 12629.

10. A biologically pure culture of Streptomyces s.p. NCIB 12629 or a natural or artificial mutant thereof, capable of producing AB-011 antibiotics in isolated amounts by means of the controlled aerobic fermentation in an aqueous nutrient medium comprising assimilatable sources of carbon and nitrogen as well as inorganic salts.

11. Use of a compound selected from AB-011 antibiotics, particularly of antibiotic AB-011 a and/or antibiotic AB-011 b, as fungicide for phytopathogenous fungi.

12. Fungicide compositions containing as their active ingredient a compound selected from AB-011 antibiotics, particularly antibiotic AB-011 a and/or antibiotic AB-011b, together with inert solid or liquid carriers, and, optionally, other additives.

## Claims (Claims for the following Contracting State(s) : ES)

1. Process for the preparation of AB-011 antibiotics, comprising the cultivation of Streptomyces s.p. NCIB 12629, or of a corresponding mutant thereof, under conditions of controlled aerobic fermentation in an aqueous nutrient medium containing assimilatable sources of carbon and nitrogen as well as inorganic salts, until antibiotic activity is obtained, the subsequent recovery of said antibiotics by means of per se known methods, and, optionally, the separation of the main components called Antibiotic AB-011 a and Antibiotic AB-011 b.

2. Process according to claim 1, in which the fermentation is carried out at a temperature of from 25 _{°} C to 30_{°}C.

3. Process according to any one of claims 1 and 2, in which the fermentation is carried out at a pH within the range of from 5 to 9.

4. Process according to any one of claims 1 to 3, in which the AB-011 antibiotics are isolated from the fermentation broth by means of filtration and subsequent use of chromatographic techniques.

5. Process according to any one of claims 1 to 4, in which the antibiotics AB-011 a and AB-01 1 b are isolated by reverse-phase chromatography on a silica-gel column using a linear elution gradient of from 50% to 80% of methanol in the corresponding mixture with water containing 25 mM/I of KH₂P0₄ and 7 mM/I of (CH₃)₄ NCI.

6. Process according to any one of claims 1 to 5 wherein the Antibiotic AB-011 a is a solid substance characterized by:
(a) the following approximate elemental analysis: 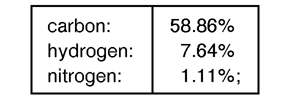
(b) a molecular weight of about 1,197.65;
(c) UV-absorbance maxima of:
2.269 at 350.4 nm; 2.197 at 332.6 nm; 1.403 at 317.3 nm; 0.679 at 303.3 nm; 0.118 at 381.1 nm and 0.097 at 405.6 nm at a concentration of 0.029 mg/ml in methanol;
(d) IR-absorbance maxima at 3421, 2960, 2930, 2855, 2035, 1718, 1634, 1570, 1448, 1403, 1383, 1340, 1302, 1268, 1168, 1063, 1036, 1009, 989, 906, 848, 794, 575, 526 and 473 cm-¹;
(e) main peaks in its ¹H-NMR spectrum at
δTMS (ppm) : 6.45-6.10* (m, 8H); 5.92 (m, 1 H); 5.69 (m, 1 H); 5.41 (m, 2H); 5.15 (m, 1 H), 4.92 (m, 1 H); 4.77-4.22 (m, 6H); 4.22-3.45* (m, 8-10H); 3.37-3.07* (m, 6-7H); 3.02 (t,1 H); 2.93 (t, 1 H); 2.88-2.72* (m, 3H); 2.45-2.27* (m, 3-4H); 2.27-2.05* (m, 3H); 2.05-1.85* (m, 3H); 1.85-1.66* (m, 1 H); 1.66-1.15* (m, 30-33H); 1.10 (d, 3H); 0.99 (d, 3H); 0.90 (m, 6H);
(f) main peaks in its ¹³C-NMR spectrum at
δTMS (ppm) : 208.6 (s); 176.3 (s); 170.3 (s); 135.8 (d); 134.6 (d); 133.5 (d); 133.2 (d); 132.9 (d); 132.7 (d); 131.7 (d); 131.4 (d); 130.0 (d); 129.5 (d); 100.3 (d); 99.8 (d); 97.4 (s); 97.3 (d); 87.1 (d); 84.4 (d); 79.9 (d); 75.0 (d); 73.5 (d); 73.1 (d); 72.1 (d); 70.7 (d); 70.0 (d); 69.0 (d); 67.1 (d); 66.1 (d); 65.9 (d); 64.0 (d); 58.1 (d); 56.9 (q); 56.3 (d); 51.6 (t); 50.5 (t); 44.7 (t); 43.0 (t); 42.5 (t); 39.7 (d); 39.4 (d); 39.0 (t); 37.5 (t); 36.3 (t); 34.4 (t); 32.1 (t); 31.7 (t); 29.3 (t); 18.0 (q); 17.9 (q); 17.1 (q); 16.0 (q); 11.5 (q);
(g) Rₜ values obtained by thin-layer chromatography (TLC) on plates of the type 60F 254 (Merck-Schuchardt) of:
0.25 in an ethanol/dioxane/30% aqueous ammonia/water (8:1:1:1) mixture;
0.0 in a methylene chloride/methanol (17:3) mixture; and
Rₜ values obtained by reverse-phase chromatography on plates of the type RP-18F 254 (Merck-Schuchardt) of:
0.29 in a methanol/aqueous solution containing 25 mM monobasic potassium phosphate + 7 mM tetramethyl-ammonium chloride (8:2) mixture;
0.44 in a methanol/acetonitrile/aqueous solution containing 25 mM monobasic potassium phosphate + 7 mM tetramethyl ammonium chloride (4:4:2) mixture;
0.13 in a methanol/10 mM aqueous solution of monobasic ammonium phosphate adjusted with phosphoric acid to pH 7.5 (8:2) mixture;
0.44 in a methanol/acetonitrile/10 mM aqueous solution of monobasic ammonium phosphate adjusted with phosphoric acid to pH 7.5 (4:4:2) mixture;
(h) a retention time (Rₜ) of about 7 minutes in reverse-phase HPLC on a Hibar Li-ChroCARTO Li-Chrosorb RP-18 column (forecolumn: Guard Pak@ RCSS C 18) when eluting with a methanol/acetonitrile/aqueous solution containing 25 mM of monobasic potassium phosphate + 7 nM tetramethyl-ammonium chloride (4:4:2) mixture at a flow rate of 0.8 ml/minute and at 40 °C;
(i) high solubility in dimethylsulfoxide and ethanol/water (1:1 V/V) or methanol/water (1:1 V/V) mixtures, scarce solubility in water and fairly high solubility in ethanol and methanol.

7. Process according to any one of claims 1 to 6 wherein the Antibiotic AB-011 b is a solid substance characterized by:
(a) the following approximate elemental analysis: 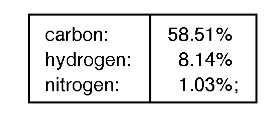
(b) a molecular weight of about 1,181;
(c) UV-absorbance maxima of:
2.872 at 349.9 nm; 2.747 at 332.4 nm; 1.999 at 316.9 nm; 0.987 at 303.3 nm; at a concentration of 0.04 mg/ml in methanol;
(d) IR-absorbance maxima at:
3415, 2926, 2855, 2060, 1721, 1634, 1568, 1450, 1406, 1383, 1302, 1264, 1190, 1168, 1063, 1036, 1007, 988, 906, 847, 804, 722, 664, 618, 576, 509, 471, 446 cm-¹;
(e) main peaks in its ¹H-NMR spectrum at:
δTMS (ppm) : 6.45-6.05 (m, 8H); 5.93 (m, 1 H); 5.70 (m, 1 H); 5.40 (m, 2H); 5.13 (m, 1 H); 4.73-4.30 (m, 6H); 4.27-3.45* (m, 8-9H); 3.37-3.09* (m, 6-7H); 3.02 (t, 1 H); 2.94 (t, 1 H); 2.75-2.60* (m, 1 H); 2.44-2.28* (m, 3H); 2.28-1.81 (m, 7-8H); 1.81-1.47* (m, 5H); 1.47-1.16* (m, 30-33H); 1.1 (d, 3H); 0.99 (d, 3H); 0.91 (m, 6H);
(f) main peaks in its ¹³C-NMR spectrum at
6TMS (ppm) : 208.7 (s); 176.3 (s); 174.9 (s); 170.4 (s); 135.7 (d); 134.7 (d); 133.5 (d); 133.3 (d); 132.9 (d); 132.7 (d); 131.7 (d); 131.5 (d); 130.0 (d); 129.5 (d); 103.3 (d); 100.0 (d); 97.4 (s); 96.9 (s); 87.2 (d); 84.5 (d); 80.0 (d); 75.1 (d); 74.9 (d); 73.1 (d); 72.2 (d); 70.7 (d); 70.0 (d); 69.0 (d); 68.5 (d); 67.8 (d); 67.1 (d); 65.9 (d); 65.7 (d); 63.9 (d); 58.1 (d); 56.9 (q); 56.2 (d); 51.5 (t); 50.7 (t); 46.5 (t); 44.7 (t); 42.5 (t); 39.1 (t); 38.5 (t); 38.0 (t); 36.3 (t); 34.1 (t); 32.3 (t); 31.8 (t); 31.6 (t); 29.3 (t); 18.1 (q); 18.0 (q); 17.9 (q); 17.4 (q); 16.3 (q); 11.6 (q);
(g) Rₜ values obtained by thin-layer chromatography (TLC) on plates of the type 60F 254 (Merck-Schuchardt) of:
0.32 in an ethanol/dioxane/30% aqueous ammonia/water (8:1:1:1) mixture;
0.00 in a methylene chloride/methanol (17:3) mixture; and
Rₜ values obtained by reverse-phase chromatography on plates of the type RP-18F 254 (Merck-Schuchardt) of:
0.22 in a methanol/aqueous solution containing 25 mM monobasic potassium phosphate + 7 mM tetramethyl-ammonium chloride (8:2) mixture;
0.35 in a methanol/acetonitrile/aqueous solution containing 25 mM monobasic potassium phosphate + 7 mM tetramethyl-ammonium chloride (4:4:2) mixture;
0.08 in a methanol/10 mM aqueous solution of monobasic ammonium phosphate adjusted with phosphoric acid to pH 7.5 (8:2) mixture;
0.20 in a methanol/acetonitrile/10 mM aqueous solution of monobasic ammonium phosphate adjusted with phosphoric acid to pH 7.5 (4:4:2) mixture;
(h) a retention time (Rₜ) of about 9 minutes in reverse-phase HPLC on a Hibar Li-ChroCART Li-Chrosorb@ RP18 column (forecolumn: Guard Pak@ RCSS C 18) when eluting with a methanol/acetonitrile/aqueous solution containing 25 mM monobasic potassium phosphate +7 mM tetramethyl-ammonium chloride (4:4:2) mixture at a flow rate of 0.8 ml/minute and at 40 °C.

8. Microorganism Streptomyces s.p., NCIB 12629.

9. A biologically pure culture of Streptomyces s.p. NCIB 12629 or a natural or artificial mutant thereof, capable of producing AB-011 antibiotics in isolated amounts by means of the controlled aerobic fermentation in an aqueous nutrient medium comprising assimilatable sources of carbon and nitrogen as well as inorganic salts.

10. Use of a compound selected from AB-011 antibiotics, particularly of Antibiotic AB-011 a and/or Antibiotic AB-011 b, as fungicide for phytopathogenous fungi.

11. Fungicide compositions containing as their active ingredient a compound selected from AB-011 antibiotics, particularly antibiotic AB-011 a and/or antibiotic AB-011 b, together with inert solid or liquid carriers, and, optionally, other additives.

## Patentansprüche (Patentansprüche für folgende(n) Vertragsstaat(en) : AT, BE, CH, DE, FR, GB, LI, NL, SE)

1. Antibiotikum AB-011 a, ein Feststoff, der gekennzeichnet ist durch:
(a) die folgende angenäherte Elementaranalyse: 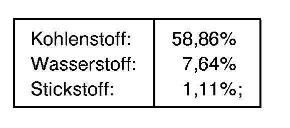
(b) ein Molekulargewicht von etwa 1.197,65;
(c) UV-Absorptionsmaxima von:
2,269 bei 350,4 nm; 2,197 bei 332,6 nm; 1,403 bei 317,3 nm; 0,679 bei 303,3 nm; 0,118 bei 381,1 nm und 0,097 bei 405,6 nm bei einer Konzentration von 0,029 mg/ml in Methanol;
(d) IR-Absorptionsmaxima bei 3421, 2960, 2930, 2855, 2035, 1718, 1634, 1570, 1448, 1403, 1383, 1340, 1302, 1268, 1168, 1063, 1036, 1009, 989, 906, 848, 794, 575, 526 und 473 cm⁻¹;
(e) Hauptpeaks in dessen ¹H-NMR-Spektrum bei
δTMS (ppm): 6,45-6,10* (m, 8H); 5,92 (m, 1 H); 5,69 (m, 1 H); 5,41 (m, 2H); 5,15 (m, 1 H), 4,92 (m, 1 H; 4,77-4,22 (m, 6H); 4,22-3,45* (m, 8-10H); 3,37-3,07* (m, 6-7H); 3,02 (t, 1 H); 2,93 (t, 1 H); 2,88-2,72* (m, 3H); 2,45-2,27* (m, 3-4H); 2,27-2,05* (m, 3H); 2,05-1,85* (m, 3H); 1,85-1,66* (m, 1H); 1,66-1,15* (m, 30-33H); 1,10 (d, 3H); 0,99 (d, 3H); 0,90 (m, 6H);
(f) Hauptpeaks in dessen ¹³C-NMR-Spektrum bei
δTMS (ppm) : 208,6 (s); 176,3 (s); 170,3 (s); 135,8 (d);134,6 (d); 133,5 (d); 133,2 (d); 132,9 (d); 132,7 (d); 131,7 (d); 131,4 (d); 130,0 (d); 129,5 (d); 100,3 (d); 99,8 (d); 97,4 (s); 97,3 (d); 87,1 (d); 84,4 (d); 79,9 (d); 75,0 (d); 73,5 (d); 73,1 (d); 72,1 (d); 70,7 (d); 70,0 (d); 69,0 (d); 67,1 (d); 66,1 (d); 65,9 (d); 64,0 (d); 58,1 (d); 56,9 (q); 56,3 (d); 51,6 (t); 50,5 (t); 44,7 (t); 43,0 (t); 42,5 (t); 39,7 (d); 39,4 (d); 39,0 (t); 37,5 (t); 36,3 (t); 34,4 (t); 32,1 (t); 31,7 (t); 29,3 (t); 18,0 (q); 17,9 (q); 17,1 (q); 16,0 (q); 11,5 (q);
(g) bei Dünnschichtchromatographie (TLC) auf Platten des Typs 60F 254 (Merck-Schuchardt) erhaltene R_{f}-Werte von:
0,25 in einer (8:1:1:1)-Mischung von Ethanol/Dioxan/wäßriger Ammoniak/Wasser;
0,0 in einer (17:3)-Mischung von Methylenchlorid/Methanol; und
bei "reversed phase" Chromatographie auf Platten des Typs RP-18F 254 (Merck-Schuchardt) erhaltene R_{f}-Werte von:
0,29 in einer (8:2)-Mischung von Methanol/wäßriger Lösung, die 25 mM einbasisches Kaliumphosphat + 7 mM Tetramethylammoniumchlorid enthält;
0,44 in einer (4:4:2)-Mischung von Methanol/Acetonitril/wäßriger Lösung, die 25 mM einbasisches Kaliumphosphat + 7 mM Tetramethylammoniumchlorid enthält;
0,13 in einer (8:2)-Mischung von Methanol/10 mM wäßriger Lösung von mit Phosphorsäure auf pH 7,5 eingestelltem einbasischem Ammoniumphosphat;
0,44 in einer (4:4:2)-Mischung von Methanol/Acetonitril/10 mM wäßriger Lösung von mit Phosphorsaure auf pH 7,5 eingestelltem einbasischem Ammoniumphosphat;
(h) eine Retentionszeit (Rₜ) von etwa 7 Minuten bei "reversed phase"-HPLC auf einer Hibar Li-ChroCART® Li-Chrosorb RP-18-Säule (Vorsäule: Guard Pak@ RCSS C 18) bei Elution mit einer (4:4:2)-Mischung von Methanol/Acetonitril/wäßriger Lösung, die 25 mM einbasisches Kaliumphosphat + 7 mM Tetramethylammoniumchlorid enthält, bei einer Flußrate von 0,8 ml/Minute und bei 40 °C;
(i) hohe Löslichkeit in Dimethylsulfoxid und Ethanol/Wasser (1:1 V/V)- oder Methanol/Wasser (1:1 v/v)-Mischungen, sehr geringe Löslichkeit in Wasser und einigermaßen hohe Löslichkeit in Ethanol und Methanol.

2. Antibiotikum AB-011 b, ein Feststoff, der gekennzeichnet ist durch:
(a) die folgende angenäherte Elementaranalyse: 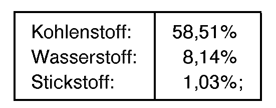
(b) ein Molekulargewicht von etwa 1.181;
(c) UV-Absorptionsmaxima von:
2,872 bei 349,9 nm; 2,747 bei 332,4 nm; 1,999 bei 316,9 nm; 0,987 bei 303,3 nm; bei einer Konzentration von 0,04 mg/ml in Methanol;
(d) IR-Absorptionsmaxima bei:
3415, 2926, 2855, 2060, 1721, 1634, 1568, 1450, 1406, 1383, 1302, 1264, 1190, 1168, 1063, 1036, 1007, 988, 906, 847, 804, 722, 664, 618, 576, 509, 471, 446 cm⁻¹;
(e) Hauptpeaks in dessen ¹H-NMR-Spektrum bei:
δTMS (ppm) : 6,45-6,05 (m, 8H); 5,93 (m, 1 H); 5,70 (m, 1 H); 5,40 (m, 2H); 5,13 (m, 1 H); 4,73-4,30 (m, 6H); 4,27-3,45* (m, 8-9H); 3,37-3,09* (m, 6-7H); 3,02 (t, 1 H); 2,94 (t, 1 H); 2,75-2,60* (m, 1 H); 2,44-2,28* (m, 3H); 2,28-1,81* (m, 7-8H); 1,81-1,47* (m, 5H); 1,47-1,16* (m, 30-33H); 1,1 (d, 3H); 0,99 (d, 3H); 0,91 (m, 6H);
(f) Hauptpeaks in dessen ¹³C-NMR-Spektrum bei:
δTMS (ppm) : 208,7 (s); 176,3 (s); 174,9 (s); 170,4 (s); 135,7 (d); 134,7 (d); 133,5 (d); 133,3 (d); 132,9 (d); 132,7 (d); 131,7 (d); 131,5 (d); 130,0 (d); 129,5 (d); 103,3 (d); 100,0 (d); 97,4 (s); 96,9 (s); 87,2 (d); 84,5 (d); 80,0 (d); 75,1 (d); 74,9 (d); 73,1 (d); 72,2 (d); 70,7 (d); 70,0 (d); 69,0 (d); 68,5 (d); 67,8 (d); 67,1 (d); 65,9 (d); 65,7 (d); 63,9 (d); 58,1 (d); 56,9 (q); 56,2 (d); 51,5 (t); 50,7 (t); 46,5 (t); 44,7 (t); 42,5 (t); 39,1 (t); 38,5 (t); 38,0 (t); 36,3 (t); 34,1 (t); 32,3 (t); 31,8 (t); 31,6 (t); 29,3 (t); 18,1 (q); 18,0 (q); 17,9 (q); 17,4 (q); 16,3 (q); 11,6 (q);
(g) bei Dünnschichtchromatographie (TLC) auf Platten des Typs 60F 254 (Merck-Schuchardt) erhaltene R_{f}-Werte von:
0,32 in einer (8:1:1:1)-Mischung von Ethanol/Dioxan/wäßriger Ammoniak/Wasser;
0,00 in einer (17:3)-Mischung von Methylenchlorid/Methanol; und
bei "reversed phase" Chromatographie auf Platten des Typs RP-18F 254 (Merck-Schuchardt) erhaltene R_{f}-Werte von:
0,22 in einer (8:2)-Mischung von Methanol/wäßriger Lösung, die 25 mM einbasisches Kaliumphosphat + 7 mM Tetramethylammoniumchlorid enthält;
0,35 in einer (4:4:2)-Mischung von Methanol/Acetonitril/wäßriger Lösung, die 25 mM einbasisches Kaliumphosphat + 7 mM Tetramethylammoniumchlorid enthält;
0,08 in einer (8:2)-Mischung von Methanol/10 mM wäßriger Lösung von mit Phosphorsäure auf pH 7,5 eingestelltem einbasischem Ammoniumphosphat;
0,20 in einer (4:4:2)-Mischung von Methanol/Acetonitril/10 mM wäßriger Lösung von mit Phosphorsäure auf pH 7,5 eingestelltem einbasischem Ammoniumphosphat;
(h) eine Retentionszeit (Rₜ) von etwa 9 Minuten bei "reversed phase"-HPLC auf einer Hibar Li-ChroCART® Li-Chrosorb RP18-Säule (Vorsäule: Guard Pak@ RCSS C 18) bei Elution mit einer (4:4:2)-Mischung von Methanol/Acetonitril/wäßriger Lösung, die 25 mM einbasisches Kaliumphosphat + 7 mM Tetramethylammoniumchlorid enthält, bei einer Flußrate von 0,8 ml/Minute und bei 40 °C.

3. AB-011-Antibiotika, erhältlich durch die kontrollierte Züchtung unter aeroben Bedingungen von Streptomyces s.p. NCIB 12629, oder einer äquivalenten Mutante davon, in einem wäßrigen Züchtungsnährmedium, das Quellen von Kohlenstoff und Stickstoff und anorganische Salze enthält, wobei die Antibiotika im wesentlichen aus dem Antibiotikum AB-011 a und dem Antibiotikum AB-011 b, wie in den Ansprüchen 1 und 2 definiert, bestehen.

4. Verfahren zur Herstellung von AB-011-Antibiotika, umfassend die Züchtung von Streptomyces s.p. NCIB 12629, oder einer entsprechenden Mutante davon, unter Bedingungen kontrollierter aerober Fermentation in einem wäßrigen Nährmedium, das assimilierbare Quellen von Kohlenstoff und Stickstoff sowie anorganische Salze enthält, bis antibiotische Aktivität erhalten wird, die nachfolgende Gewinnung der Antibiotika mittels an sich bekannter Verfahren und gegebenenfalls die Auftrennung der Hauptkomponenten, dem Antibiotikum AB-011 und dem Antibiotikum AB-011b, wie sie in den Ansprüchen und 2 definiert sind.

5. Verfahren nach Anspruch 4, worin die Fermentation bei einer Temperatur von 25°C bis 30°C durchgeführt wird.

6. Verfahren nach irgendeinem der Ansprüche 4 und 5, worin die Fermentation bei einem pH im Bereich von 5 bis 9 durchgeführt wird.

7. Verfahren nach irgendeinem der Ansprüche 4 bis 6, worin die AB-011 Antibiotika mittels Filtration und nachfolgender Anwendung chromatographischer Techniken aus der Fermentationsbrühe isoliert werden.

8. Verfahren nach irgendeinem der Ansprüche 4 bis 7, worin die Antibiotika AB-011 a und AB-01 1 b durch "reversed phase" -Chromatographie auf einer Silicagel-Säule isoliert werden, unter Verwendung eines linearen Elutionsgradienten von 50% bis 80% Methanol in der entsprechenden Mischung mit Wasser, das 25 mM/I KH₂P0₄ und 7 mM/I (CH₃)₄NCI enthält.

9. Mikroorganismus Streptomyces s.p., NCIB 12629.

10. Biologisch reine Kultur von Streptomyces s.p. NCIB 12629 oder einer natürlichen oder künstlichen Mutante davon, die imstande ist, AB-011-Antibiotika in isolierten Mengen mittels der kontrollierten aeroben Fermentation in einem wäßrigen Nährmedium zu erzeugen, das assimilierbare Quellen von Kohlenstoff und Stickstoff sowie anorganische Salze enthält.

11. Verwendung einer Verbindung, die aus AB-011-Antibiotika, insbesondere dem Antibiotikum AB-011a und/oder dem Antibiotikum AB-011b, ausgewählt ist, als Fungizid für phytopathogene Pilze.

12. Fungizide Zusammensetzungen, die als deren aktiven Bestandteil eine Verbindung enthalten, die aus AB-011-Antibiotika, insbesondere dem Antibiotika AB-011 a und/oder dem Antibiotikum AB-011b, ausgewählt ist, zusammen mit inerten festen oder flüssigen Trägern und gegebenenfalls anderen Zusätzen.

## Patentansprüche (Patentansprüche für folgende(n) Vertragsstaat(en) : ES)

1. Verfahren zur Herstellung von AB-011-Antibiotika, umfassend die Züchtung von Streptomyces s.p. NCIB 12629, oder einer entsprechenden Mutante davon, unter Bedingungen kontrollierter aerober Fermentation in einem wäßrigen Nährmedium, das assimilierbare Quellen von Kohlenstoff und Stickstoff sowie anorganische Salze enthält, bis antibiotische Aktivität erhalten wird, die nachfolgende Gewinnung der Antibiotika mittels an sich bekannter Verfahren und gegebenenfalls die Auftrennung der Hauptkomponenten, die als Antibiotikum AB-011 a und Antibiotikum AB-01 1 b bezeichnet werden.

2. Verfahren nach Anspruch 1, worin die Fermentation bei einer Temperatur von 25°C bis 30°C durchgeführt wird.

3. Verfahren nach irgendeinem der Ansprüche 1 und 2, worin die Fermentation bei einem pH im Bereich von 5 bis 9 durchgeführt wird.

4. Verfahren nach irgendeinem der Ansprüche 1 bis 3, worin die AB-011 Antibiotika mittels Filtration und nachfolgender Anwendung chromatographischer Techniken aus der Fermentationsbrühe isoliert werden.

5. Verfahren nach irgendeinem der Ansprüche 1 bis 4, worin die Antibiotika AB-011 a und AB-01 1 b durch "reversed phase" -Chromatographie auf einer Silicagel-Säule isoliert werden, unter Verwendung eines linearen Elutionsgradienten von 50% bis 80% Methanol in der entsprechenden Mischung mit Wasser, das 25 mM/I KH₂P0₄ und 7 mM/I (CH₃)₄NCI enthält.

6. Verfahren nach irgendeinem der Ansprüche 1 bis 5, worin das Antibiotikum AB-011 ein Feststoff ist, der gekennzeichnet ist durch:
(a) die folgende angenäherte Elementaranalyse: 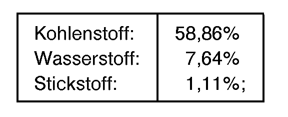
(b) ein Molekulargewicht von etwa 1.197,65;
(c) UV-Absorptionsmaxima von:
2,269 bei 350,4 nm; 2,197 bei 332,6 nm; 1,403 bei 317,3 nm; 0,679 bei 303,3 nm; 0,118 bei 381,1 nm und 0,097 bei 405,6 nm bei einer Konzentration von 0,029 mg/ml in Methanol;
(d) IR-Absorptionsmaxima bei 3421, 2960, 2930, 2855, 2035, 1718, 1634, 1570, 1448, 1403, 1383, 1340, 1302, 1268, 1168, 1063, 1036, 1009, 989, 906, 848, 794, 575, 526 und 473 cm⁻¹;
(e) Hauptpeaks in dessen ¹H-NMR-Spektrum bei
δTMS (ppm): 6,45-6,10* (m, 8H); 5,92 (m, 1 H); 5,69 (m, 1 H); 5,41 (m, 2H); 5,15 (m, 1 H), 4,92 (m, 1 H; 4,77-4,22 (m, 6H); 4,22-3,45* (m, 8-10H); 3,37-3,07* (m, 6-7H); 3,02 (t, 1 H); 2,93 (t, 1 H); 2,88-2,72* (m, 3H); 2,45-2,27* (m, 3-4H); 2,27-2,05* (m, 3H); 2,05-1,85* (m, 3H); 1,85-1,66* (m, 1H); 1,66-1,15* (m, 30-33H); 1,10 (d, 3H); 0,99 (d, 3H); 0,90 (m, 6H);
(f) Hauptpeaks in dessen ¹³C-NMR-Spektrum bei
δTMS (ppm): 208,6 (s); 176,3 (s); 170,3 (s); 135,8 (d);134,6 (d); 133,5 (d); 133,2 (d); 132,9 (d); 132,7 (d); 131,7 (d); 131,4 (d); 130,0 (d); 129,5 (d); 100,3 (d); 99,8 (d); 97,4 (s); 97,3 (d); 87,1 (d); 84,4 (d); 79,9 (d); 75,0 (d); 73,5 (d); 73,1 (d); 72,1 (d); 70,7 (d); 70,0 (d); 69,0 (d); 67,1 (d); 66,1 (d); 65,9 (d); 64,0 (d); 58,1 (d); 56,9 (q); 56,3 (d); 51,6 (t); 50,5 (t); 44,7 (t); 43,0 (t); 42,5 (t); 39,7 (d); 39,4 (d); 39,0 (t); 37,5 (t); 36,3 (t); 34,4 (t); 32,1 (t); 31,7 (t); 29,3 (t); 18,0 (q); 17,9 (q); 17,1 (q); 16,0 (q); 11,5 (q);
(g) bei Dünnschichtchromatographie (TLC) auf Platten des Typs 60F 254 (Merck-Schuchardt) erhaltene R_{f}-Werte von:
0,25 in einer (8:1:1:1)-Mischung von Ethanol/Dioxan/wäßriger Ammoniak/Wasser;
0,0 in einer (17:3)-Mischung von Methylenchlorid/Methanol; und
bei "reversed phase" Chromatographie auf Platten des Typs RP-18F 254 (Merck-Schuchardt) erhaltene R_{f}-Werte von:
0,29 in einer (8:2)-Mischung von Methanol/wäßriger Lösung, die 25 mM einbasisches Kaliumphosphat + 7 mM Tetramethylammoniumchlorid enthält;
0,44 in einer (4:4:2)-Mischung von Methanol/Acetonitril/wäßriger Lösung, die 25 mM einbasisches Kaliumphosphat + 7 mM Tetramethylammoniumchlorid enthält;
0,13 in einer (8:2)-Mischung von Methanol/10 mM wäßriger Lösung von mit Phosphorsäure auf pH 7,5 eingestelltem einbasischem Ammoniumphosphat;
0,44 in einer (4:4:2)Mischung von Methanol/Acetonitril/10 mM wäßriger Lösung von mit Phosphorsäure auf pH 7,5 eingestelltem einbasischem Ammoniumphosphat;
(h) eine Retentionszeit (Rₜ) von etwa 7 Minuten bei "reversed phase"-HPLC auf einer Hibar Li-ChroCART® Li-Chrosorb RP-18-Säule (Vorsäule: Guard Pak@ RCSS C 18) bei Elution mit einer (4:4:2)-Mischung von Methanol/Acetonitril/wäßriger Lösung, die 25 mM einbasisches Kaliumphosphat + 7 mM Tetramethylammoniumchlorid enthält, bei einer Flußrate von 0,8 ml/Minute und bei 40 °C;
(i) hohe Löslichkeit in Dimethylsulfoxid und Ethanol/Wasser (1:1 V/V)- oder Methanol/Wasser (1:1 V/V)-Mischungen, sehr geringe Löslichkeit in Wasser und einigermaßen hohe Löslichkeit in Ethanol und Methanol.

7. Verfahren nach irgendeinem der Ansprüche 1 bis 6, worin das Antibiotikum AB-011 ein Feststoff ist, der gekennzeichnet ist durch:
(a) die folgende angenäherte Elementaranalyse: 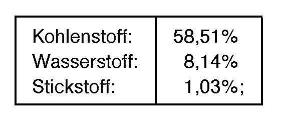
(b) ein Molekulargewicht von etwa 1.181;
(c) UV-Absorptionsmaxima von:
2,872 bei 349,9 nm; 2,747 bei 332,4 nm; 1,999 bei 316,9 nm; 0,987 bei 303,3 nm; bei einer Konzentration von 0,04 mg/ml in Methanol;
(d) IR-Absorptionsmaxima bei:
3415, 2926, 2855, 2060, 1721, 1634, 1568, 1450, 1406, 1383, 1302, 1264, 1190, 1168, 1063, 1036, 1007, 988, 906, 847, 804, 722, 664, 618, 576, 509, 471, 446 cm⁻¹;
(e) Hauptpeaks in dessen ¹H-NMR-Spektrum bei:
δTMS (ppm): 6,45-6,05 (m, 8H); 5,93 (m, 1 H); 5,70 (m, 1 H); 5,40 (m, 2H); 5,13 (m, 1H); 4,73-4,30 (m, 6H); 4,27-3,45* (m, 8-9H); 3,37-3,09* (m, 6-7H); 3,02 (t, 1 H); 2,94 (t, 1 H); 2,75-2,60* (m, 1 H); 2,44-2,28* (m, 3H); 2,28-1,81* (m, 7-8H); 1,81-1,47* (m, 5H); 1,47-1,16* (m, 30-33H); 1,1 (d, 3H); 0,99 (d, 3H); 0,91 (m, 6H);
(f) Hauptpeaks in dessen ¹³C-NMR-Spektrum bei:
δTMS (ppm): 208,7 (s); 176,3 (s); 174,9 (s); 170,4 (s); 135,7 (d); 134,7 (d); 133,5 (d); 133,3 (d); 132,9 (d); 132,7 (d); 131,7 (d); 131,5 (d); 130,0 (d); 129,5 (d); 103,3 (d); 100,0 (d); 97,4 (s); 96,9 (s); 87,2 (d); 84,5 (d); 80,0 (d); 75,1 (d); 74,9 (d); 73,1 (d); 72,2 (d); 70,7 (d); 70,0 (d); 69,0 (d); 68,5 (d); 67,8 (d); 67,1 (d); 65,9 (d); 65,7 (d); 63,9 (d); 58,1 (d); 56,9 (q); 56,2 (d); 51,5 (t); 50,7 (t); 46,5 (t); 44,7 (t); 42,5 (t); 39,1 (t); 38,5 (t); 38,0 (t); 36,3 (t); 34,1 (t); 32,3 (t); 31,8 (t); 31,6 (t); 29,3 (t); 18,1 (q); 18,0 (q); 17,9 (q); 17,4 (q); 16,3 (q); 11,6 (q);
(g) bei Dünnschichtchromatographie (TLC) auf Platten des Typs 60F 254 (Merck-Schuchardt) erhaltene R_{f}-Werte von:
0,32 in einer (8:1:1:1)-Mischung von Ethanol/Dioxan/wäßriger Ammoniak/Wasser;
0,00 in einer (17:3)-Mischung von Methylenchlorid/Methanol; und
bei "reversed phase" Chromatographie auf Platten des Typs RP-18F 254 (Merck-Schuchardt) erhaltene R_{f}-Werte von:
0,22 in einer (8:2)-Mischung von Methanol/wäßriger Lösung, die 25 mM einbasisches Kaliumphosphat + 7 mM Tetramethylammoniumchlorid enthält;
0,35 in einer (4:4:2)-Mischung von Methanol/Acetonitril/wäßriger Lösung, die 25 mM einbasisches Kaliumphosphat + 7 mM Tetramethylammoniumchlorid enthält;
0,08 in einer (8:2)-Mischung von Methanol/10 mM wäßriger Lösung von mit Phosphorsäure auf pH 7,5 eingestelltem einbasischem Ammoniumphosphat;
0,20 in einer (4:4:2)-Mischung von Methanol/Acetonitril/10 mM wäßriger Lösung von mit Phosphorsäure auf pH 7,5 eingestelltem einbasischem Ammoniumphosphat;
(h) eine Retentionszeit (Rₜ) von etwa 9 Minuten bei "reversed phase"-HPLC auf einer Hibar Li- ChroCART@ Li-Chrosorb RP18-Säule (Vorsäule: Guard Pak@ RCSS C 18) bei Elution mit einer (4:4:2)-Mischung von Methanol/Acetonitril/wäßriger Lösung, die 25 mM einbasisches Kaliumphosphat + 7 mM Tetramethylammoniumchlorid enthält, bei einer Flußrate von 0,8 ml/Minute und bei 40 °C.

8. Mikroorganismus Streptomyces s.p., NCIB 12629.

9. Biologisch reine Kultur von Streptomyces s.p. NCIB 12629 oder einer natürlichen oder künstlichen Mutante davon, die imstande ist, AB-011-Antibiotika in isolierten Mengen mittels der kontrollierten aeroben Fermentation in einem wäßrigen Nährmedium zu erzeugen, das assimilierbare Quellen von Kohlenstoff und Stickstoff sowie anorganische Salze enthält.

10. Verwendung einer Verbindung, die aus AB-011-Antibiotika, insbesondere dem Antibiotikum AB-011a und/oder dem Antibiotikum AB-011 b, ausgewählt ist, als Fungizid für phytopathogene Pilze.

11. Fungizide Zusammensetzungen, die als deren aktiven Bestandteil eine Verbindung enthalten, die aus AB-011-Antibiotika, insbesondere dem Antibiotika AB-011 a und/oder dem Antibiotikum AB-011 b, ausgewählt ist, zusammen mit inerten festen oder flüssigen Trägern und gegebenenfalls anderen Zusätzen.

## Revendications (Revendications pour l'(les) Etat(s) contractant(s) suivant(s) : AT, BE, CH, DE, FR, GB, LI, NL, SE)

1. Antibiotique AB-011 a consistant en une substance solide caractérisée par:
(a) l'analyse élémentaire approximative suivante: 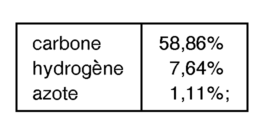
(b) un poids moléculaire d'environ 1197,65;
(c) des maxima d'absorbance dans l'UV de:
2,269 à 350,4 nm; 2,197 à 332,6 nm; 1,403 à 317,3 nm; 0,679 à 303,3 nm; 0,118 à 381,1 nm et 0,097 à 405,6 nm, à une concentration de 0,029 mg/ml dans du méthanol;
(d) des maxima d'absorbance dans l'infrarouge à (cm⁻¹):
3421, 2960, 2930, 2855, 2035, 1718, 1634, 1570, 1448, 1403, 1383, 1340, 1302, 1268, 1168, 1063, 1036, 1009, 989, 906, 848, 794, 575, 526, 473;
(e) des pics principaux dans son spectre ¹H-RMN à:
δTMS (ppm) : 6,45-6,10* (m, 8H); 5,92 (m, 1 H); 5,69 (m, 1 H); 5,41 (m, 2H); 5,15 (m, 1 H); 4,92 (m, 1 H); 4,77-4,22 (m, 6H); 4,22-3,45* (m, 8-10H); 3,37-3,07* (m, 6-7H); 3,02 (t, 1 H); 2,93 (t, 1 H); 2,88-2,72* (m,3H); 2,45-2,27* (m, 3-4H); 2,27-2,05* (m,3H); 2,05-1,85* (m, 3H); 1,85-1,66* (m, 1 H); 1,66-1,15* (m, 30-33H); 1,10 (d, 3H); 0,99 (d, 3H); 0,90 (m, 6H).
(f) des pics principaux dans son spectre ¹³C-RMN à:
δTMS (ppm): 208,6 (s); 176,3 (s); 170,3 (s); 135,8 (d); 134,6 (d); 133,5 (d); 133,2 (d); 132,9 (d); 132,7 (d); 131,7 (d); 131,4 (d); 130,0 (d); 129,5 (d); 100,3 (d); 99,8 (d); 97,4 (s); 97,3 (d); 87,1 (d); 84,4 (d); 79,9 (d); 75,0 (d); 73,5 (d); 73,1 (d); 72,1 (d); 70,7 (d); 70,0 (d); 69,0 (d); 67,1 (d); 66,1 (d); 65,9 (d); 64,0 (d); 58,1 (d); 56,9 (q); 56,3 (d); 51,6 (t); 50,5 (t); 44,7 (t); 43,0 (t); 42,5 (t); 39,7 (d); 39,4 (d); 39,0 (t); 37,5 (t); 36,3 (t); 34,4 (t); 32,1 (t); 31,7 (t); 29,3 (t); 18,0 (q); 17,9 (q); 17,1 (q); 16,0 (q); 11,5 (q).
(g) des valeurs R_{f} obtenues par chromatographie sur couche mince (TLC) sur des plaques de type 60F 254 (Merck-Schuchardt) de:
0,25 dans un mélange éthanol/dioxane/solution aqueuse d'ammoniaque (à 30%)/eau (8/1/1/1);
0,0 dans un mélange de chlorure de méthylène/méthanol (17/3); et
des valeurs R_{f} obtenues par chromatographie en phase inverse sur des plaques de type RP-18F 254 (Merck-Schuchardt) de:
0,29 dans un mélange de méthanol/solution aqueuse contenant 25 mM de monohydrogénophosphate de potassium + 7 mM de chlorure de tétraméthyl-ammonium (8/2);
0,44 dans un mélange de méthanol/acétonitrile/solutio n aqueuse contenant 25 mM de monohydrogénophosphate de potassium + 7 mM de chlorure de tétraméthyl-ammonium (4/4/2);
0,13 dans un mélange de méthanol/solution aqueuse de monohydrogénophosphate d'ammonium à 10 mM dont le pH est ajusté à 7,5 avec de l'acide phosphorique (8/2);
0,44 dans un mélange de méthanol/acétonitrile/solution aqueuse de monohydrogénophosphate d'ammonium à 10 mM dont le pH est ajusté à 7,5 avec de l'acide phosphorique (4/4/2);
(h) un temps de rétention (Rₜ) d'environ 7 minutes en HPLC en phase inverse sur une colonne Hibar Li-chroCART® Li-Chrosorb RP-18, (précolonne: Guard Pak@ RCSS C18) lorsqu'elle est éluée avec un mélange méthanol/acétonitrile/solution aqueuse contenant 25 mM de monohydrogénophosphate de potassium + 7 mM de chlorure de tétraméthylammonium (4/4/2) à un débit de 0,8 ml/minute et à 40°C;
(i) une solubilité élevée dans le diméthylsulfoxyde et dans les mélanges éthanol/eau (1/1 v/v) ou méthanol/eau (1/1 v/v), une faible solubilité dans l'eau et une assez bonne solubilité dans l'éthanol et le méthanol.

2. Antibiotique AB-011 b, consistant en une substance solide caractérisée par:
(a) l'analyse élémentaire approximative suivante: 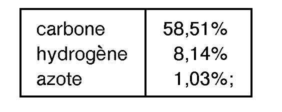
(b) un poids moléculaire d'environ 1181;
(c) des maxima d'absorbance dans l'UV de:
2,872 à 349,9 nm; 2,747 à 332,4 nm; 1,999 à 316,9 nm; 0,987 à 303,3 nm; déterminé à une concentration de 0,04 mg/ml dans le méthanol;
(d) des maxima d'absorbance dans l'infrarouge à (cm-1) de:
3415, 2926, 2855, 2060, 1721, 1634, 1568, 1450, 1406, 1383, 1302, 1264, 1190, 1168, 1063, 1036, 1007, 988, 906, 847, 804, 722, 664, 618, 576, 509, 471, 446;
(e) des pics principaux dans son spectre ¹H-RMN à:
δTMS (ppm): 6,45-6,05 (m, 8H); 5,93 (m, 1 H); 5,70 (m, 1 H); 5,40 (m, 2H); 5,13 (m, 1 H); 4,73-4,30 (m, 6H); 4,27-3,45* (m, 8-9H); 3,37-3,09* (m, 6-7H); 3,02 (t, 1 H); 2,94 (t, 1 H); 2,75-2,60* (m, 1 H); 2,44-2,28* (m, 3H); 2,28-1,81 (m, 7-8H); 1,81-1,47* (m, 5H); 1,47-1,16* (m, 30-33H); 1,1 (d, 3H); 0,99 (d, 3H); 0,91 (m, 6H).
(f) des pics principaux dans son spectre ¹³C-RMN à:
δTMS (ppm) : 208,7 (s); 176,3 (s); 174,9 (s); 170,4 (s); 135,7 (d); 134,7 (d); 133,5 (d); 133,3 (d); 132,9 (d); 132,7 (d); 131,7 (d); 131,5 (d); 130,0 (d); 129,5 (d); 103,3 (d); 100,0 (d); 97,4 (s); 96,9 (d); 87,2 (d); 84,5 (d); 80,0 (d); 75,1 (d); 74,9 (d); 73,1 (d); 72,2 (d); 70,7 (d); 70,0 (d); 69,0 (d); 68,5 (d); 67,8 (d); 67,1 (d); 65,9 (d); 65,7 (d); 63,9 (d); 58,1 (d); 56,9 (q); 56,2 (d); 51,5 (t); 50,7 (t); 46,5 (t); 44,7 (t); 42,5 (t); 39,1 (t); 38,5 (t); 38,0 (t); 36,3 (t); 34,1 (t); 32,3 (t); 31,8 (t); 31,6 (t); 29,3 (t); 18,1 (q); 18,0 (q); 17,9 (q); 17,4 (q); 16,3 (q); 11,6 (q).
(g) des valeurs R_{f} obtenues par chromatographie sur couche mince (TLC) sur des plaques de type 60F 254 (Merck Schuchardt) de:
0,32 dans un mélange éthanol/dioxane/solution aqueuse d'ammoniaque (à 30%)/eau (8/1/1/1);
0,00 dans un mélange de chlorure de méthylène/méthanol (17/3); et
des valeurs R_{f} obtenues par chromatographie en phase inverse sur des plaques de type RP-18F 254 (Merck-Schuchardt) de:
0,22 dans un mélange de méthanol/solution aqueuse contenant 25 mM de monohydrogénophosphate de potassium + 7 mM de chlorure de tétraméthyl-ammonium (8/2);
0,35 dans un mélange méthanol/acétonitrile/solution aqueuse contenant 25 mM de monohydrogénophosphate de potassium + 7 mM de chlorure de tétraméthylammonium (4/4/2);
0,08 dans un mélange méthanol/solution aqueuse de monohydrogénophosphate d'ammonium à 10 mM dont le pH est ajusté à 7,5 avec de l'acide phosphorique (8/2);
0,20 dans un mélange méthanol/acétonitrile/solution aqueuse de monohydrogénophosphate d'ammonium à 10 mM dont le pH est ajusté à 7,5 avec de l'acide phosphorique (4/4/2);
(h) un temps de rétention (Rₜ) de 9 minutes en HPLC en phase inverse sur une colonne Hibar Li- chroCART@ Li-Chrosorb@ RP-18, (précolonne: Guard Pak@ RCSS C18) lorsqu'elle est éluée avec un mélange méthanol/acétonitrile/solution aqueuse contenant 25 mM de monohydrogénophosphate de potassium + 7 mM de chlorure de tétraméthylammonium (4/4/2) à un débit de 0,8 ml/minute et à 40_{°}C;

3. Antibiotiques AB-011, susceptibles d'être obtenus au moyen d'une culture contrôlée dans les conditions aérobies de Streptomyces s.p. NCIB 12629 ou d'un mutant équivalent ou dérivant, dans un milieu de culture nutritif aqueux contenant des sources de carbone, d'azote et de sels inorganiques, lesdits antibiotiques étant sensiblement composés de l'antibiotique AB-011 a et de l'antibiotique AB-011 b tels que définis dans les revendications 1 et 2.

4. Procédé de préparation des antibiotiques AB-011 comprenant la culture de Streptomyces s.p. NCIB 12629 ou d'un mutant correspondant en dérivant, dans des conditions de fermentation aérobie contrôlées dans un milieu nutritif aqueux contenant des sources assimilables de carbone et d'azote ainsi que des sels inorganiques jusqu'à ce qu'une activité antibiotique soit obtenue, la récupération ultérieure desdits antibiotiques au moyen de méthodes connues per se et éventuellement la séparation des composants principaux, désignés AB-011 a et AB-011 b, tels que définis dans les revendications 1 et 2.

5. Procédé selon la revendication 4, dans lequel la fermentation est mise en oeuvre à une température de 25 à 30 _{°} C.

6. Procédé selon l'une quelconque des revendications 4 et 5, dans lequel la fermentation est mise en oeuvre à un pH compris entre 5 et 9.

7. Procédé selon l'une quelconque des revendications 4 à 6, dans lequel les antibiotiques AB-011 sont isolés à partir d'un bouillon de fermentation par filtration puis par utilisation des techniques chromatographiques.

8. Procédé selon l'une quelconque des revendications 4 à 7, dans lequel les antibiotiques AB-011 a et AB-011 b sont isolés par chromatographie en phase inverse sur une colonne de gel de silice en utilisant un gradient d'élution linéaire de 50 à 80% de méthanol dans le mélange correspondant avec de l'eau contenant 25 mM/I de KH₂PO₄ et 7 mM/I de (CH₃)₄NCI.

9. Microorganisme Streptomyces s.p. NCIB 12629.

10. Une culture biologiquement pure de Streptomyces s.p. NCIB 12629 ou d'un mutant naturel ou de synthèse en dérivant, capable de produire les antibiotiques AB-011,
en des quantités susceptibles d'être isolées, au moyen d'une fermentation aérobie contrôlée dans un milieu nutritif aqueux comprenant des sources assimilables de carbone et d'azote ainsi que des sels inorganiques.

11. Utilisation d'un dérivé sélectionné parmi les antibiotiques AB-011, particulièrement de l'antibiotique AB-011 a et/ou de l'antibiotique AB-011 b, en tant que fongicide pour des champignons phytopathogènes.

12. Compositions fongicides contenant en tant que principe actif, un composé sélectionné parmi les antibiotiques AB-011, en particulier l'antibiotique AB-011 a et/ou l'antibiotique AB-011 b, ainsi que des supports liquides ou solides inertes et éventuellement d'autres additifs.

## Revendications (Revendications pour l'(les) Etat(s) contractant(s) suivant(s) : ES)

1. Procédé de préparation des antibiotiques AB-011 comprenant la culture de Streptomyces s.p. NCIB 12629 ou d'un mutant correspondant en dérivant, dans des conditions de fermentation aérobie contrôlées dans un milieu nutritif aqueux contenant des sources assimilables de carbone et d'azote ainsi que des sels inorganiques, jusqu'à ce qu'une activité antibiotique soit obtenue, la récupération ultérieure desdits antibiotiques au moyen de méthodes connues per se et éventuellement la séparation des composants principaux, désignés antibiotique AB-011 a et antibiotique AB-011 b.

2. Procédé selon la revendication 1, dans lequel la fermentation est mise en oeuvre à une température de 25 à 30°C.

3. Procédé selon l'une quelconque des revendications 1 et 2, dans lequel la fermentation est mise en oeuvre à un pH compris entre 5 et 9.

4. Procédé selon l'une quelconque des revendications 1 à 3, dans lequel les antibiotiques AB-011 sont isolés à partir du bouillon de fermentation par des moyens de filtration puis par utilisation de techniques chromatographiques.

5. Procédé selon l'une quelconque des revendications 1 à 4, dans lequel les antibiotiques AB-011 a et AB-011 b sont isolés par chromatographie en phase inverse sur une colonne de gel de silice en utilisant un gradient d'élution linéaire de 50 à 80% de méthanol dans le mélange correspondant avec de l'eau contenant 25 mM/I de KH₂PO₄ et 7 mM/I de (CH₃)₄NCI.

6. Procédé selon l'une quelconque des revendications 1 à 5, dans lequel l'antibiotique AB-011 a est une substance solide caractérisée par:
(a) l'analyse élémentaire approximative suivante: 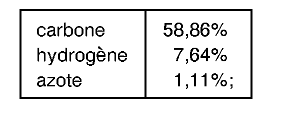
(b) un poids moléculaire d'environ 1197,65;
(c) des maxima d'absorbance dans l'UV de:
2,269 à 350,4 nm; 2,197 à 332,6 nm; 1,403 à 317,3 nm; 0,679 à 303,3 nm; 0,118 à 381,1 nm et 0,097 à 405,6 nm, à une concentration de 0,029 mg/ml dans du méthanol;
(d) des maxima d'absorbance dans l'infrarouge à (cm⁻¹):
3421, 2960, 2930, 2855, 2035, 1718, 1634, 1570, 1448, 1403, 1383, 1340, 1302, 1268, 1168, 1063, 1036, 1009, 989, 906, 848, 794, 575, 526, 473;
(e) des pics principaux dans son spectre ¹H-RMN à:
5TMS (ppm): 6,45-6,10^{*} (m, 8H); 5,92 (m, 1 H); 5,69 (m, 1 H); 5,41 (m, 2H); 5,15 (m, 1 H); 4,92 (m, 1 H); 4,77-4,22 (m, 6H); 4,22-3,45* (m, 8-10H); 3,37-3,07* (m, 6-7H); 3,02 (t, 1 H); 2,93 (t, 1 H); 2,88-2,72* (m,3H); 2,45-2,27* (m, 3-4H); 2,27-2,05* (m,3H); 2,05-1,85* (m, 3H); 1,85-1,66* (m, 1 H); 1,66-1,15* (m, 30-33H); 1,10 (d, 3H); 0,99 (d, 3H); 0,90 (m, 6H).
(f) des pics principaux dans son spectre ¹³C-RMN à:
OTMS (ppm): 208,6 (s); 176,3 (s); 170,3 (s); 135,8 (d); 134,6 (d); 133,5 (d); 133,2 (d); 132,9 (d); 132,7 (d); 131,7 (d); 131,4 (d); 130,0 (d); 129,5 (d); 100,3 (d); 99,8 (d); 97,4 (s); 97,3 (d); 87,1 (d); 84,4 (d); 79,9 (d); 75,0 (d); 73,5 (d); 73,1 (d); 72,1 (d); 70,7 (d); 70,0 (d); 69,0 (d); 67,1 (d); 66,1 (d); 65,9 (d); 64,0 (d); 58,1 (d); 56,9 (q); 56,3 (d); 51,6 (t); 50,5 (t); 44,7 (t); 43,0 (t); 42,5 (t); 39,7 (d); 39,4 (d); 39,0 (t); 37,5 (t); 36,3 (t); 34,4 (t); 32,1 (t); 31,7 (t); 29,3 (t); 18,0 (q); 17,9 (q); 17,1 (q); 16,0 (q); 11,5 (q).
(g) des valeurs R_{f} obtenues par chromatographie sur couche mince (TLC) sur des plaques de type 60F 254 (Merck-Schuchardt) de:
0,25 dans un mélange éthanol/dioxane/solution aqueuse d'ammoniaque (à 30%)/eau (8/1/1/1);
0,0 dans un mélange de chlorure de méthylène/méthanol (17/3); et
des valeurs R_{f} obtenues par chromatographie en phase inverse sur des plaques de type RP-18F 254 (Merck-Schuchardt) de:
0,29 dans un mélange de méthanol/solution aqueuse contenant 25 mM de monohydrogénophosphate de potassium + 7 mM de chlorure de tétraméthyl-ammonium (8/2);
0,44 dans un mélange de méthanol/acétonitrile/solution aqueuse contenant 25 mM de monohydrogénophosphate de potassium + 7 mM de chlorure de tétraméthyl-ammonium (4/4/2);
0,13 dans un mélange de méthanol/solution aqueuse de monohydrogénophosphate d'ammonium à 10 mM dont le pH est ajusté à 7,5 avec de l'acide phosphorique (8/2);
0,44 dans un mélange de méthanol/acétonitrile/solution aqueuse de monohydrogénophosphate d'ammonium à 10 mM dont le pH est ajusté à 7,5 avec de l'acide phosphorique (4/4/2);
(h) un temps de rétention (Rₜ) d'environ 7 minutes en HPLC en phase inverse sur une colonne Hibar Li-ChroCART® Li-Chrosorb RP-18, (précolonne: Guard Pak@ RCSS C18) lorsqu'elle est éluée avec un mélange méthanol/acétonitrile/solution aqueuse contenant 25 mM de monohydrogénophosphate de potassium + 7 mM de chlorure de tétraméthylammonium (4/4/2) à un débit de 0,8 ml/minute et à 40°C;
(i) une solubilité élevée dans le diméthylsulfoxyde et dans les mélanges éthanol/eau (1/1 v/v) ou méthanol/eau (1/1 v/v), une faible solubilité dans l'eau et une assez bonne solubilité dans l'éthanol et le méthanol.

7. Procédé selon l'une quelconque des revendications 1 à 6, dans lequel l'antibiotique AB-011 b, est une substance solide caractérisée par:
(a) l'analyse élémentaire approximative suivante: 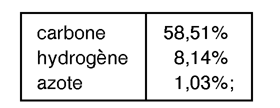
(b) un poids moléculaire d'environ 1181;
(c) des maxima d'absorbance dans l'UV de:
2,872 à 349,9 nm; 2,747 à 332,4 nm; 1,999 à 316,9 nm; 0,987 à 303,3 nm; déterminé à une concentration de 0,04 mg/ml dans le méthanol;
(d) des maxima d'absorbance dans l'infrarouge à (cm-1) de:
3415, 2926, 2855, 2060, 1721, 1634, 1568, 1450, 1406, 1383, 1302, 1264, 1190, 1168, 1063, 1036, 1007, 988, 906, 847, 804, 722, 664, 618, 576, 509, 471, 446;
(e) des pics principaux dans son spectre ¹H-RMN à:
δTMS (ppm): 6,45-6,05 (m, 8H); 5,93 (m, 1 H); 5,70 (m, 1 H); 5,40 (m, 2H); 5,13 (m, 1 H); 4,73-4,30 (m, 6H); 4,27-3,45* (m, 8-9H); 3,37-3,09* (m, 6-7H); 3,02 (t, 1 H); 2,94 (t, 1 H); 2,75-2,60* (m, 1 H); 2,44-2,28* (m, 3H); 2,28-1,81 (m, 7-8H); 1,81-1,47* (m, 5H); 1,47-1,16* (m, 30-33H); 1,1 (d, 3H); 0,99 (d, 3H); 0,91 (m, 6H).
(f) des pics principaux dans son spectre ¹³C-RMN à:
δTMS (ppm) : 208,7 (s); 176,3 (s); 174,9 (s); 170,4 (s); 135,7 (d); 134,7 (d); 133,5 (d); 133,3 (d); 132,9 (d); 132,7 (d); 131,7 (d); 131,5 (d); 130,0 (d); 129,5 (d); 103,3 (d); 100,0 (d); 97,4 (s); 96,9 (d); 87,2 (d); 84,5 (d); 80,0 (d); 75,1 (d); 74,9 (d); 73,1 (d); 72,2 (d); 70,7 (d); 70,0 (d); 69,0 (d); 68,5 (d); 67,8 (d); 67,1 (d); 65,9 (d); 65,7 (d); 63,9 (d); 58,1 (d); 56,9 (q); 56,2 (d); 51,5 (t); 50,7 (t); 46,5 (t); 44,7 (t); 42,5 (t); 39,1 (t); 38,5 (t); 38,0 (t); 36,3 (t); 34,1 (t); 32,3 (t); 31,8 (t); 31,6 (t); 29,3 (t); 18,1 (q); 18,0 (q); 17,9 (q); 17,4 (q); 16,3 (q); 11,6 (q).
(g) des valeurs R_{f} obtenues par chromatographie sur couche mince (TLC) sur des plaques de type 60F 254 (Merck Schuchardt) de:
0,32 dans un mélange éthanol/dioxane/solution aqueuse d'ammoniaque (à 30%)/eau (8/1/1/1);
0,00 dans un mélange de chlorure de méthylène/méthanol (17/3); et
des valeurs R_{f} obtenues par chromatographie en phase inverse sur des plaques de type RP-18F 254 (Merck-Schuchardt) de:
0,22 dans un mélange de méthanol/solution aqueuse contenant 25 mM de monohydrogénophosphate de potassium + 7 mM de chlorure de tétraméthyl-ammonium (8/2);
0,35 dans un mélange méthanol/acétonitrile/solution aqueuse contenant 25 mM de monohydrogénophosphate de potassium + 7 mM de chlorure de tétraméthylammonium (4/4/2);
0,08 dans un mélange méthanol/solution aqueuse de monohydrogénophosphate d'ammonium à 10 mM dont le pH est ajusté à 7,5 avec de l'acide phosphorique (8/2);
0,20 dans un mélange méthanol/acétonitrile/solution aqueuse de monohydrogénophosphate d'ammonium à 10 mM dont le pH est ajusté à 7,5 avec de l'acide phosphorique (4/4/2);
(h) un temps de rétention (Rₜ) d'environ 9 minutes en HPLC en phase inverse sur une colonne Hibar Li-chroCART® Li-Chrosorb® RP-18, (précolonne: Guard Pak@ RCSS C18) lorsqu'elle est éluée avec un mélange méthanol/acétonitrile/solution aqueuse contenant 25 mM de monohydrogénophosphate de potassium + 7 mM de chlorure de tétraméthylammonium (4/4/2) à un débit de 0,8 ml/minute et à 40°C.

8. Microorganisme Streptomyces s.p. NCIB 12629.

9. Une culture biologiquement pure de Streptomyces s.p. NCIB 12629 ou d'un mutant naturel ou de synthèse en dérivant, capable de produire les antibiotiques AB-011, en des quantités susceptibles d'être isolées, au moyen d'une fermentation aérobie contrôlée dans un milieu nutritif aqueux comprenant des sources assimilables de carbone et d'azote ainsi que des sels inorganiques.

10. Utilisation d'un composé sélectionné parmi les antibiotiques AB-011, particulièrement de l'antibiotique AB-011 a et/ou de l'antibiotique AB-011 b, en tant que fongicide pour des champignons phytopathogènes.

11. Compositions fongicides contenant en tant que principe actif, un composé sélectionné parmi les antibiotiques AB-011, en particulier l'antibiotique AB-011 a et/ou l'antibiotique AB-011 b, ainsi que des supports liquides ou solides inertes et éventuellement d'autres additifs.
